# EUROPEAN PATENT SPECIFICATION

(11) **EP 4 536 086 B1**
(45) Date of publication and mention of the grant of the patent: **04.11.2026**
(21) Application number: 23738290.8
(22) Date of filing: 06.06.2023
(51) Int. Cl.: A61B 6/00, G01R 33/56, A61M 5/00, A61M 5/172, G16H 20/40, G16H 30/40, G16H 40/63, G16H 50/20, G16H 50/70

(54) **USE OF CATH LAB IMAGES FOR PREDICTION AND CONTROL OF CONTRAST USAGE**
VERWENDUNG VON CATHLABORBILDERN ZUR VORHERSAGE UND STEUERUNG DER KONTRASTVERWENDUNG
UTILISATION D'IMAGES DE LABORATOIRE DE CATHÉTÉRISME POUR LA PRÉDICTION ET LE CONTRÔLE DE L'UTILISATION DU CONTRASTE

(30) Priority: 06.06.2022 US 202263365939 P
(43) Date of publication of application: 16.04.2025
(73) Proprietor: Medtronic Vascular Inc., Santa Rosa, CA 95403 (US); Digital Surgery Ltd, London EC1V 2QY (GB)
(72) Inventor: TEMPLETON, Kaitlin, Santa Rosa, California 95403 (US); BEHRMANN, Heidrun, Santa Rosa, California 95403 (US); BELENCHIA, Giordana, M., Boulder, Colorado 80301 (US); JOSHEE, Abiral, Santa Rosa, California 95403 (US); LEBOW, Benjamin, J., Santa Rosa, California 95403 (US); SAENZ SALAS, Federico, Santa Rosa, California 95403 (US); VU, Kevin, H., Mounds View, Minnesota 55112 (US); YU, Angela, London EC1V 2TT (GB); ZALEWSKI, Jeffrey, M., Santa Rosa, California 95403 (US); FONTANA, Jason, T., Santa Rosa, California 95403 (US); WEIDMAN, Jason, R., Santa Rosa, California 95403 (US)
(74) Representative: Strange, Harry George
(86) International application number: PCT/US2023/024607
(87) International publication number: WO 2023/239742

(56) References cited:
- CN-A- 1 839 755
- JP-A- 2010 035 831
- JP-A- 2020 032 246
- JP-B2- 6 791 664
- US-A1- 2001 056 233
- US-A1- 2006 224 104

## Description

This applicant claims the benefit of US Provisional Patent Application No. 63/365,939, filed 6 June 2022.

### TECHNICAL FIELD

This disclosure relates to the use of images captured during a medical procedure.

### BACKGROUND

During a medical procedure, a clinician may use an imaging system to be able to visualize internal anatomy of a patient. Such an imaging system may display anatomy, medical instruments, or the like, and may be used to diagnose a patient condition or assist in guiding a clinician in moving a device, such as a medical instrument to an intended location inside the patient. Imaging systems may use sensors to capture video images which may be displayed during the medical procedure. Imaging systems include angiography systems, ultrasound imaging systems, computed tomography (CT) scan systems, magnetic resonance imaging (MRI) systems, isocentric C-arm fluoroscopic systems, positron emission tomography (PET) systems, intravascular ultrasound (IVUS), optical coherence tomography (OCT), as well as other imaging systems.

Document US2006224104A1 may be considered to disclose a medical system comprising: a memory; and processing circuitry communicatively coupled to the memory, the processing circuitry being configured to: determine, at a current point of a cardiac catheterization lab procedure, a cumulative amount of contrast used during the cardiac catheterization lab procedure up to the current point; determine a planned amount of contrast to have been used by the current point of the cardiac catheterization lab procedure; and output, for display and during the cardiac catheterization lab procedure a graphical representation of the cumulative amount of contrast used.

### SUMMARY

In general, this disclosure is directed to various techniques and medical systems for using images captured during a medical procedure for procedure and/or device evaluation. For example, a system may track the motion of a medical instrument to assess operator technique as a variable for research, such as which operator techniques may provide the best outcomes. As used herein a medical instrument includes any device which may be used to treat a patient. The system may also connect other pieces of equipment commonly used in the cardiac catheterization laboratory (Cath Lab) and integrate their respective data, such as integrating ablation data from a renal denervation (RDN) generator to an imaging system, such as fluoroscopy, to enable a more detailed evaluation of outcomes. Additionally, or alternatively, the system may track the motion of a device to assess cross-ability of that device with a particular type of lesion. By tracking the location of a medical instrument during a therapeutic medical procedure, a system may determine which operator techniques may provide the best outcome and/or which medical instruments may provide the best outcomes, for example, for a particular type of lesion. The system may include a computer vision model that may be used to identify, classify, and/or score a particular lesion. The system may also include a machine learning model that may be used to determine potential treatments having the greatest chances at successful outcomes and may present such potential treatment options to a clinician before, during or after a therapeutic medical procedure.

This disclosure is also directed to various techniques and medical systems for using images captured during a diagnostic medical procedure for treatment planning purposes. For example, during a diagnostic session (e.g., a diagnostic angiogram), there are three possible outcomes. First, is that a clinician may determine no intervention is necessary. Second, a clinician may determine that an urgent intervention is necessary and that the clinician can handle the intervention during the same Cath Lab session (e.g., without the patient leaving and coming back another time). Third, treatment may be required, but either the clinician is uncomfortable performing the treatment or the hospital in which the Cath Lab is located does not have the necessary equipment to perform the treatment. In the case of the third possible outcome, imaging data (e.g., angiogram data) from the diagnostic medical procedure may exist. An example system may use such imaging data to plan or assist a clinician in planning the treatment. Such a system may include a computer vision model and a machine learning model. The computer vision model may be used to identify, classify, and/or score a particular lesion. The machine learning model may be used to determine potential treatments having the greatest chances at successful outcomes and may present such potential treatment strategies to a clinician to plan treatment for a therapeutic medical procedure. The system may be configured to run simulations on potential treatment strategies to assist the clinician in selecting one or more treatment strategies to use during the therapeutic medical procedure.

This disclosure is also directed various techniques and medical systems for streaming or sharing a representation of imaging data from one or more image sensors during a medical procedure (e.g., a cardiac catheterization medical procedure) with a remote clinician. For instance, while a particular clinician is performing a medical procedure in a cardiac catheterization laboratory (Cath Lab), a medical system may establish (e.g., via a secure network) a communication session with a device associated with a remote clinician (i.e., a second clinician not located within the Cath Lab). The computing system may stream or share the imaging data via a communication session to the remote clinician. In this way, the medical system may enable the remote clinician to provide assistance (e.g., view and/or consult) to the particular clinician (i.e., the clinician actually performing the procedure) as the particular clinician performs the procedure. Enabling such remote assistance may present one or more advantages. As one example, remote assistance may improve the particular clinician's comfort and/or confidence in a particular diagnosis, treatment strategy, technique, equipment or tool selection, or the like. As such, a consultation from a remote clinician via a communication session may result in more cases moving from the third possible outcome mentioned above, in which treatment must be delayed, into the second possible outcome mentioned above, where the operating clinician handles the intervention during the same session. Enabling remote assistance may provide the aforementioned benefits without the burdens of having to obtain on-site assistance from another clinician. For instance, the particular clinician may be located at a rural medical facility and may be the only clinician on-site with Cath Lab experience. The techniques of this disclosure enable such a clinician to obtain live intra-procedure assistance without requiring another clinician to travel to the rural facility. Although described primarily with respect to medical procedures which include percutaneous coronary intervention (PCI) procedures, medical systems according to the present disclosure apply to other medical procedures within the Cath Lab. For example, medical systems according to the present disclosure may be used to conduct one or more other medical procedures carried out in the Cath lab including diagnostic cardiac catheterization, atrial septal atherectomy, cardiac ablation, cardiac resynchronization therapy, CardioMEMS^{™} HF System Implant, coronary stenting, coronary ultrasound, electrophysiology studies, implantable cardioverter defibrillator (ICD) placement, implantable loop recorder, intravascular ultrasound (IVUS), percutaneous transluminal angioplasty (PTCA), peripheral angioplasty, permanent pacemaker placement, rotoblator, TAVR, three-dimensional mapping, valvuloplasty, or the like. In some examples, a PCI procedure is a medical procedure conducted on a patient with a lesion (e.g., a bifurcated lesion) within their vasculature. As described herein, examples of the present disclosure relate to medical systems for treatment where the patient condition is a lesion. However, other patient conditions which may be treated within the Cath lab are considered, for example structural heart conditions(e.g., cardio myopathy, congential heart disease, heart valve disease, or the like).

In some examples, a medical system according to the present disclosure may prevent streaming or sharing a patient's personal health information (PHI) with a clinician who does not have permission to view PHI. For example, the system may be configured to determine a permission state for the remote clinician, who may be within the same hospital or network or outside the hospital or network associated with the operating clinician. In some examples, responsive to a determination that the permission state of the remote clinician is "outside" or some other designation indicating that the remote clinician is not affiliated with the Cath Lab where the intervention is being performed, the medical system may be configured to redact personal health information from the representation of the imaging data. In this way, personal health information (PHI) may be protected, and the benefits of clinician consultation while both clinicians are viewing the imaging data may be realized (e.g., without having to obtain further patient consent for PHI disclosure). The systems and techniques of the present disclosure may allow for advice or instruction from the remote clinician viewing the imaging data in real time through the communication session. Accordingly, because the patient may only need to undergo a single intervention, risks may be reduced because fewer interventions may be made, and the patient may receive a necessary treatment without delay.

This disclosure is also directed to medical systems and techniques for generating and/or sharing curated video highlights or images from a medical procedure. Medical systems according to the present disclosure may generate a condensed version of imaging data (e.g., fluoroscopy imaging) sensed by one or more image sensors during a medical procedure. The condensed version of the imaging data may include images corresponding to particular events during a medical procedure, such as a cardiac catheterization medical procedure. In some examples, medical systems according to the present disclosure may be configured receive user input to begin or end a video excerpt, which may correspond to a key portions of a medical procedure. Additionally, or alternatively, medical systems according to execute a computer vision model to recognize a patient condition or an intraprocedural event in received imaging data. In some examples, responsive to recognizing a patient condition or intraprocedural event, the medical system may be configured to present an option to a clinician to provide user input to begin the video excerpt of the received imaging data. In some examples, the medical system may be configured to redact personal health information from the condensed version of the imaging data. In some examples, the medical system may be further configured to share or stream the condensed version of the imaging data with a remote clinician. Thus, systems and techniques according to the present disclosure may allow for easy sharing, via a secure platform, of identified events or before/after images of a medical procedure. Additionally, in some examples, the remote clinician may view the condensed version of the procedure to quickly review a medical procedure in process.

This disclosure is also directed to medical systems and techniques for receiving imaging data from one or more image sensors which includes one or more identifying elements and generating a de-identified version of the imaging data which does not include one or more identifying elements. In some examples, at least one of the one or more identifying elements may be personal health information (PHI), which may be protected health information as defined by HIPAA or a similar regulation. The medical system may be configured to redact, remove, obfuscate, or otherwise render illegible text information such as a patient name, birthdate, or other personal health information. In some examples, imaging data from one or more image sensors may include PHI, and the medical system may be configured to scan the imaging data, identify a text overlay, and redact, remove, obfuscate, or otherwise render illegible the text overlay. In some examples, the medical system may be further configured to upload the de-identified version of the imaging data to a server. In some examples, the medical system may be further configured to present a clinician an option to post the de-identified version of the imaging data on a social network or otherwise share the de-identified version of the imaging data. In some examples, the medical system may be configured to prevent or block the imaging data from being posted or published to a social network before it has been properly de-identified. In some examples, the social network may be a physician-only social network (e.g., Murmur). Relative to hospitals lacking a secure way to share or relying on third-party anonymizing software, medical systems and techniques according to the present disclosure may allow a secure way to post and discuss case video, video highlights, before/after images, and the like. Medical systems and techniques according to the present disclosure may facilitate clinician discussion and education and/or boost the reputation of an operating clinician who is able to elegantly and safely share imaging data taken from a medical procedure (e.g., a cardiac catheterization medical procedure) that they have performed.

This disclosure is also directed to medical systems and techniques for clinician education. In some examples, medical systems and techniques according to the present disclosure may allow a clinician to see how their case or treatment strategy differed from the strategy of another clinician (e.g., an expert) in a similar case. In some examples, medical systems according to the present disclosure may be configured to output for display an overlay or side-by-side representation of imaging data from a first medical procedure and second medical procedure stored in a memory. The medical system may execute a computer vision model and/or a machine learning model to use propensity matching to identify a second medical procedure to output for display with the first medical procedure based on the similarity of one or more patient conditions of the first medical procedure with the second medical procedure. In some examples, the patient condition may be a lesion, and the medical system may use propensity matching by comparing one or more lesion characteristics from a lesion associated with the first medical procedure, and identify and select the individual medical procedure from the plurality of medical procedures stored in the memory which includes a similar lesion (e.g., the most similar lesion) based on the one or more lesion characteristics to output for display as the second medical procedure. In some examples, the medical system may be further configured to allow a clinician to sort and/or filter the medical procedures stored in the memory in one or more ways, to allow the clinician to filter the desired results. Accordingly, because medical systems of the present disclosure may use computer vision and/or machine learning to find and output for display a similar medical procedure stored in the memory for comparison to the instant medical procedure, the clinician may receive targeted feedback more relevant than could otherwise be viewed. In this way, medical systems according to the present disclosure may provide desirable learning and education benefits which may upskill a clinician and better prepare them to perform an upcoming medical procedure.

This disclosure is also directed to a learning pathway for a clinician (e.g., an interventional cardiologist) to complete in exchange for credit. In some examples, the credit may be integrated with a credentialling body to provide an elegant procedure for reporting and receiving credits from a credentialling body. In some examples, the medical system may be configured to capture user information from a user interacting with the medical system to identify the user. The medical system may be configured to store data representative of an excerpt of imaging data from an individual medical procedure of a plurality of cardiac catheterization medical procedures in the memory. The excerpt from the individual medical procedure may be output for display and, subsequent to outputting for display the excerpt from the medical procedure, the medical system may credit the user with watching the excerpt of the medical procedure. In some examples, the medical system may be further configured to award at least a portion of a continuing medical education (CME) credit to a user. In some examples, the medical system may be configured to present the user an option to filter by any one of a plurality of accepted medical techniques (e.g., accepted percutaneous coronary intervention techniques). Conventionally, certain types of clinicians may lack a structured curriculum for progressing past a certain point in their career. A medical system according to the present disclosure may provide a mechanism for setting up an online curriculum. Such a mechanism may provide for further specialization of a clinician, because the clinician may watch and receive credit for watching a plurality of medical procedures. For example, medical systems according to the present disclosure may help an interventional cardiologist further specialize in complex PCI (e.g., bifurcation disease) by watching a plurality of medical procedures relating to one or more of the six currently accepted techniques for treating a lesion.

This disclosure is also directed to monitoring and providing guidance for contrast usage during Cath Lab procedures. The amount of contrast used is a balance between using enough such that images contain necessary detail and not using too much so as to cause undesirable side effects. In some examples, a system may track how much contrast has been used and provide a clinician with guidance. For instance, the system may inform the clinician as to how the actual amount of contrast used compares with an expected/predicted amount of contrast.

Aspects of this disclosure are applicable to at least Cath Lab procedures. Example Cath lab procedures include, but are not necessarily limited to, coronary procedures, renal denervation (RDN) procedures, structural heart and aortic (SH&A) procedures (e.g., transcatheter aortic valve replacement (TAVR), transcatheter mitral valve replacement (TMVR), and the like), device implantation procedures (e.g., heart monitors, pacemakers, defibrillators, and the like), etc.

In one example, a medical system includes memory configured to store at least one computer vision model; and processing circuitry communicatively coupled to the memory, the processing circuitry being configured to: receive imaging data of at least a portion of a vasculature of a patient generated during a cardiac catheterization procedure; and execute the at least one computer vision model to determine characteristics of a lesion of the vasculature based on the received imaging data.

In another example, a method includes receiving, by processing circuitry, imaging data of at least a portion of a vasculature of a patient generated during a cardiac catheterization procedure; and executing, by the processing circuitry, at least one computer vision model to determine characteristics of a lesion in the vasculature based on the received imaging data.

In another example, a non-transitory computer readable medium stores instructions, which, when executed, cause processing circuitry to receive imaging data of at least a portion of a vasculature of a patient generated during a cardiac catheterization procedure; and execute at least one computer vision model to determine characteristics of a lesion in the vasculature based on the received imaging data.

In another example, a medical system includes memory configured to store at least one computer vision model and at least one machine learning model; and processing circuitry communicatively coupled to the memory, the processing circuitry being configured to: receive diagnostic imaging data of at least a portion of a vasculature of a patient generated during a cardiac diagnostic procedure; execute the at least one computer vision model to determine characteristics of a lesion in the vasculature based on the received diagnostic imaging data; and execute the at least one machine learning model to determine at least one treatment strategy based on the determined characteristic of the lesion, the at least one treatment strategy comprising at least one treatment technique and at least one medical instrument.

In another example, a method includes receiving, by processing circuitry, diagnostic imaging data of at least a portion of a vasculature of a patient generated during a cardiac diagnostic procedure; executing, by processing circuitry, at least one computer vision model to determine characteristics of a lesion in the vasculature based on the received diagnostic imaging data; and executing, by the processing circuitry, at least one machine learning model to determine at least one treatment strategy based on the determined characteristic of the lesion, the at least one treatment strategy comprising at least one treatment technique and at least one medical instrument.

In another example, a non-transitory computer-readable storage medium stores instructions, which, when executed, cause processing circuitry to: receive diagnostic imaging data of at least a portion of a vasculature of a patient generated during a cardiac diagnostic procedure; execute at least one computer vision model to determine characteristics of a lesion in the vasculature based on the received diagnostic imaging data; and execute at least one machine learning model to determine at least one treatment strategy based on the determined characteristic of the lesion, the at least one treatment strategy comprising at least one treatment technique and at least one medical instrument.

In another example, a medical system includes a memory; one or more image sensors; and processing circuitry communicatively coupled to the memory, the processing circuitry being configured to: receive, from a first clinician performing a cardiac catheterization lab procedure, a representation of user input to request a consult from a second clinician that is located remotely from the first clinician; establish, responsive to receiving the representation of user input to request the consult, a communication session between a first computing device associated with the first clinician and a second computing device associated with the second clinician; and stream, via the communication session, a representation of data of the cardiac catheterization lab procedure captured by the one or more image sensors.

In another example a non-transitory computer-readable storage medium stores instructions, which when executed cause processing circuitry to: receive, from a first clinician that is performing a cardiac catheterization lab procedure, a representation of user input to request a consult from a second clinician that is located remotely from the first clinician; establish, responsive to receiving the representation of user input to request the consult, a communication session between a computing device associated with the first clinician and a computing device associated with the second clinician; and stream, via the communication session, a representation of data of the cardiac catheterization lab procedure captured by the one or more image sensors.

In another example, a method includes receiving, by processing circuitry, from a first clinician that is performing a cardiac catheterization lab procedure, a representation of user input to request a consult from a second clinician that is located remotely from the first clinician; establish, responsive to receiving the representation of user input to request the consult, a communication session between a computing device associated with the first clinician and a computing device associated with the second clinician; and stream, via the communication session, a representation of data of the cardiac catheterization lab procedure captured by one or more image sensors.

In another example, a medical system includes a memory; and processing circuitry communicatively coupled to the memory, the processing circuitry being configured to: receive imaging data, the imaging data sensed by one or more image sensors during a cardiac catheterization medical procedure; and generate, based on the imaging data, a condensed version of the imaging data, the condensed version of the imaging data including images corresponding to particular events of the cardiac catheterization medical procedure.

In another example, a method includes receiving, by processing circuitry, imaging data, imaging data sensed by one or more image sensors during a cardiac catheterization medical procedure; and generating, based on the imaging data, a condensed version of the imaging data, the condensed version of the imaging data including images corresponding to particular events of the cardiac catheterization medical procedure.

In another example, a medical system includes a memory; and processing circuitry communicatively coupled to the memory, the processing circuitry being configured to: receive imaging data, the imaging data sensed by one or more image sensors during a cardiac catheterization medical procedure, the received imaging data including one or more identifying elements; and generate, based on the imaging data, a de-identified version of the imaging data, the de-identified version of the imaging data not including at least one of the one or more identifying elements.

In another example, a method includes receiving, by processing circuitry, imaging data sensed by one or more image sensors during a cardiac catheterization medical procedure, the received imaging data including one or more identifying elements; and generating, by processing circuitry, based on the imaging data, a de-identified version of the imaging data, the de-identified version of the imaging data not including at least one of the one or more identifying elements.

In another example, a medical system includes a memory; and processing circuitry communicatively coupled to the memory, the processing circuitry being configured to: receive imaging data from one or more image sensors during a first cardiac catheterization medical procedure; execute at least one computer vision model to identify a second cardiac catheterization medical procedure of a plurality of cardiac catheterization medical procedures stored in the memory; and output, for display, a representation of imaging data from the first cardiac catheterization medical procedure and a representation of imaging data from the second cardiac catheterization medical procedure.

In another example, a method includes receiving, by processing circuitry imaging data from one or more image sensors during a first cardiac catheterization medical procedure; executing, by processing circuitry, at least one computer vision model to identify a second cardiac catheterization medical procedure of a plurality of cardiac catheterization medical procedures stored in a memory; and outputting, by processing circuitry, for display via a display, a representation of imaging data from the first cardiac catheterization medical procedure and a representation of imaging data from the second cardiac catheterization medical procedure.

In another example, a medical system includes a memory; and processing circuitry communicatively coupled to the memory, the processing circuitry being configured to: capture user information from a user interacting with the medical system to identify the user; store data representative of imaging data from a plurality of cardiac catheterization medical procedures in the memory; output for display data representative of an individual cardiac catheterization medical procedure of the plurality of cardiac catheterization medical procedures stored in the memory; and subsequent to outputting for display the data representative of the individual cardiac catheterization medical procedure, credit the user with watching the excerpt of the cardiac catheterization medical procedure.

In another example, capturing, by processing circuitry, user information from a user interacting with the medical system to identify the user; storing, by processing circuitry, data representative of imaging data from a plurality of cardiac catheterization medical procedures in the memory; outputting for display by a display, by processing circuitry, data representative of an individual cardiac catheterization medical procedure of the plurality of cardiac catheterization medical procedures stored in the memory; and subsequent to outputting for display the data representative of the individual cardiac catheterization medical procedure, credit the user with watching the individual cardiac catheterization medical procedure.

In another example, a medical system includes a memory; and processing circuitry communicatively coupled to the memory, the processing circuitry being configured to: determine a cumulative amount of contrast used during a cardiac catheterization lab procedure; and output, for display and during the cardiac catheterization lab procedure, a graphical representation of the cumulative amount of contrast used.

In another example, a method includes determining a cumulative amount of contrast used during a cardiac catheterization lab procedure; and outputting, for display and during the cardiac catheterization lab procedure, a graphical representation of the cumulative amount of contrast used.

In another example, a computer-readable storage medium stores instructions that cause one or more processors to determine a cumulative amount of contrast used during a cardiac catheterization lab procedure; and output, for display and during the cardiac catheterization lab procedure, a graphical representation of the cumulative amount of contrast used.

These and other aspects of the present disclosure will be apparent from the detailed description below. In no event, however, should the above summaries be construed as limitations on the claimed subject matter, which subject matter is defined solely by the attached claims.

This summary is intended to provide an overview of the subject matter described in this disclosure. It is not intended to provide an exclusive or exhaustive explanation of the apparatus and methods described in detail within the accompanying drawings and description below. Further details of one or more examples are set forth in the accompanying drawings and the description below.

### BRIEF DESCRIPTION OF DRAWINGS

FIG. 1 is a schematic perspective view of one example of a system for guiding a medical instrument through a region of a patient according to one or more aspects of this disclosure.
FIG. 2 is a block diagram of one example of a computing device in accordance with one or more aspects of this disclosure.
FIG. 3 is a block diagram of an example energy generation device in accordance with one or more aspects of this disclosure.
FIG. 4 is a conceptual diagram illustrating the overlaying of a representation of ablated tissue over imaging data in accordance with one or more aspects of this disclosure.
FIG. 5 is a flow diagram illustrating example techniques for determining characteristics of a lesion according to one or more aspects of this disclosure.
FIG. 6 is a schematic perspective view of one example of a system for determining treatment strategies according to one or more aspects of this disclosure.
FIG. 7 is a schematic view of one example of a computing device in accordance with one or more aspects of this disclosure.
FIG. 8 is a flow diagram illustrating example techniques for determining treatment strategies according to one or more aspects of this disclosure.
FIG. 9 is a schematic perspective view of one example of a system for establishing a communication system between an operating clinician and a remote clinician and streaming imaging data representative of a medical procedure according to one or more aspects of this disclosure.
FIG. 10 is a flow diagram illustrating example techniques for streaming a representation of data to a remote clinician according to one or more aspects of this disclosure.
FIG. 11 is a time diagram illustrating example condensed versions of imaging data sensed by one or more image sensors during a cardiac catheterization medical procedure according to one or more aspects of the present disclosure.
FIG. 12 is a flow diagram illustrating example techniques for generating a condensed version of imaging data sensed by one or more image sensors according to one or more aspects of the present disclosure.
FIG. 13A is an example conceptual screenshot illustrating an example representation of imaging data including identifying elements according to one or more aspects of the present disclosure.
FIG. 13B is an example screenshot illustrating an example representation of imaging data not including at least one of the identifying elements of FIG 13A.
FIG. 14 is a flow diagram illustrating example techniques for generating a de-identified version of imaging data sensed by one or more image sensors during a medical procedure according to one or more aspects of the present disclosure.
FIG. 15 is a screenshot illustrating an example user interface for using an example medical system of the present disclosure for educational purposes, according to one or more aspects of the present disclosure.
FIG. 16 is a flow diagram illustrating example techniques for using an example medical system of the present disclosure for educational purposes, according to one or more aspects of the present disclosure.
FIG. 17 is a flow diagram illustrating example techniques for using an example medical system of the present disclosure for educational purposes, according to one or more aspects of the present disclosure.
FIG. 18 is a schematic perspective view of example medical system configured to provide contrast usage data, according to one or more aspects of the present disclosure.
FIG. 19 is a conceptual diagram illustrating an example graphical user interface (GUI) that includes contrast usage data, in accordance with one or more aspects of this disclosure.
FIG. 20 is a flow diagram illustrating example techniques for providing contrast usage data, according to one or more aspects of the present disclosure.
FIG. 21 is a conceptual diagram illustrating an example machine learning model according to one or more aspects of this disclosure
FIG. 22 is a conceptual diagram illustrating an example training process for a machine learning model in accordance with one or more aspects of this disclosure.

### DETAILED DESCRIPTION

Imaging systems may be used to assist a clinician in a medical procedure, such as a diagnostic medical procedure, a therapeutic medical procedure, such as a percutaneous coronary intervention (PCI) procedure, and RDN procedure, a structural heart procedure, or the like, or any combination thereof. For example, imaging systems may be used to determine the presence of lesions within a vasculature of a patient that may be limiting or obstructing blood flow within the vasculature of the patient. Imaging systems may also be used when performing an ablation procedure, angioplasty procedure, or other therapeutic medical procedure intended to treat lesions within the vasculature (including the heart) of the patient. While described primarily herein with respect to the vasculature of a patient, imaging systems described herein may be used for other medical purposes and are not limited to cardiovascular purposes. Imaging systems may generate image and/or video data via sensors. This video data may be displayed during a medical procedure and/or be recorded for later use. The video data may include representations of portions of vasculature or heart of a patient, including one or more lesions which may be restricting blood flow through the portion of the vasculature or the heart of the patient, a geometry and location within a blood vessel or the heart of such lesions, and/or any medical instrument which may be within a field of view of one or more sensors of the imaging system. In some examples, contrasting fluid may be injected into the vasculature of the patient and the imaging data may include fluoroscopy imaging.

As referred to herein, a medical procedure may be a diagnostic medical procedure or a therapeutic medical procedure. A diagnostic medical procedure is a medical procedure in which imaging or other techniques are used to diagnose disease. A therapeutic medical procedure is a medical procedure in which therapy is delivered and/or an intervention is performed, for example, a PCI. A single Cath Lab session may include 1) only a diagnostic medical procedure, for example, where no lesion is identified that requires treatment or in which the treatment is too difficult for a given clinician or the hospital in which the Cath Lab is located does not have the necessary equipment to treat the lesion; 2) only a therapeutic medical procedure, for example, where a lesion was previously diagnosed; or 3) a diagnostic medical procedure followed by a therapeutic medical procedure. An example of a medical procedure that may be performed in a Cath Lab is a cardiac catheterization procedure (which may be a diagnostic medical procedure or a therapeutic medical procedure).

In some examples, a representation of the data from the sensor(s), gathered during a medical procedure, may be shared or streamed with a clinician located remotely (i.e., outside of the Cath Lab where the medical procedure is taking place). The remote clinician may consult or advise during the medical procedure, and the medical system may allow for this real-time input from a remote clinician, potentially improving medical outcomes. Additionally, or alternatively, imaging data from one or more sensors may be stored in a memory or uploaded to a network to be used for training or educational purposes. Medical systems according to the present disclosure may include processing circuitry configured to use computer vision and/or machine learning to use propensity matching to identify and select a medical procedure from a plurality of medical procedures stored in the memory to output for display on a user interface. Accordingly, a clinician may see how a medical procedure treating a similar patient condition (e.g., a lesion with similar size, location, geometry, or the like) was treated in a previous medical procedure.

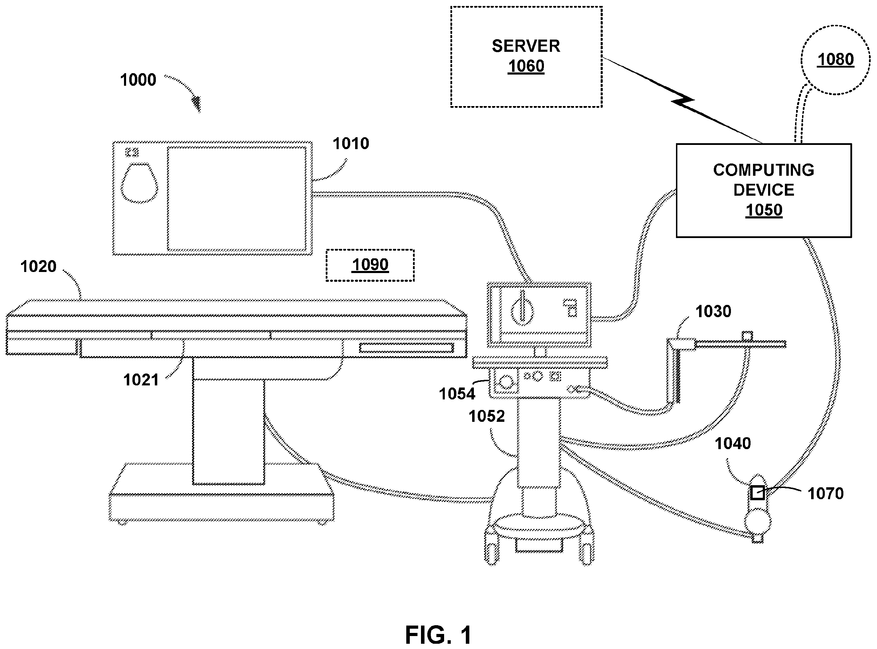

FIG. 1 is a schematic perspective view of one example of one example of a system for guiding a medical instrument through a region of a patient according to one or more aspects of this disclosure. System 1000 includes a guidance workstation 1052, a display device 1010, a table 1020, a medical instrument 1030, an imager 1040, and a computing device 1050. System 1000 may be an example of a system for use in a catheter laboratory (Cath lab). Guidance workstation 1052 may include, for example, an off-the-shelf device, such as a laptop computer, desktop computer, tablet computer, smart phone, or other similar device. In some examples, controllers of such devices may generate controller data. Such devices may include atherectomy devices, energy generation devices, or other devices which may generate data. In some examples, guidance workstation may be a specific purpose device. Guidance workstation 1052 may be configured to control an electrosurgical generator, a peristaltic pump, a power supply, or any other accessories and peripheral devices relating to, or forming part of, system 1000. In some examples, guidance workstation 1052 may include an electrosurgical generator, such as energy generation device 1054. For example, energy generation device 1054 may include an RDN generator configured to generate radiofrequency energy for an ablation catheter (e.g., medical instrument 1030) to deliver to ablate tissue in renal arteries to treat hypertension. While shown as part of guidance workstation 1052, in some examples, energy generation device 1054 may be a separate device. Computing device 1050 may include, for example, an off-the-shelf device such as a laptop computer, desktop computer, tablet computer, smart phone, or other similar device or may include a specific purpose device. In some examples, guidance workstation 1052 may perform various control functions with respect to imager 1040 and may interact extensively computing device 1050. Guidance workstation 1052 may be communicatively coupled to computing device 1050, enabling guidance workstation 1052 to control the operation of imager 1040 and receive the output of imager 1040. In some examples, computing device 1050 may control various operations of imager 1040.

Display device 1010 may be configured to output instructions, images, and messages relating to at least one of a performance, position, orientation, or trajectory of medical instrument 1030. Further, the display device 1010 may be configured to output information regarding medical instrument 1030, e.g., model number, type, size, etc. Table 1020 may be, for example, an operating table or other table suitable for use during a medical procedure that may optionally include an electromagnetic (EM) field generator 1021. EM field generator 1021 may be optionally included and used to generate an EM field during the medical procedure and, when included, may form part of an EM tracking system that is used to track the positions of one or more medical instruments within the body of a patient. EM field generator 1021 may include various components, such as a specially designed pad to be placed under, or integrated into, an operating table or patient bed.

Medical instruments may also be visualized by using imaging, such as ultrasound imaging. In the example of FIG. 1, an imager 1040, such as an intravascular ultrasound (IVUS) device, an ultrasound wand, or other imaging device, may be used to image the patient's body during the medical procedure to visualize the locations of medical instruments, such as surgical instruments, device delivery or placement devices, and implants, inside the patient's body. Imager 1040 may one or more sensors 1070. For example, imager 1040 may include an intervenes ultrasound probe having an ultrasound transducer array. In some examples, imager 1040 may include an ultrasound transducer array, including a plurality of transducer elements or other type of imaging sensors. These transducer elements may be configured to sense ultrasound energy reflected off of anatomy of the patient and/or medical instrument 1030. Imager 1040 may optionally have an EM tracking sensor embedded within or attached to an intervenes ultrasound probe, for example, as a clip-on sensor, or a sticker sensor. While described primarily as an IVUS device, imager 1040 may be any type of imaging device including one or more sensors, such as a CT device, an MRI device, a fluoroscopic device, a PET device, an angiogram device, or the like.

Imager 1040 may image a region of interest in the patient's body. The particular region of interest may be dependent on anatomy, the diagnostic medical procedure, and/or the intended therapy. For example, when performing a PCI, a portion of the vasculature may be the region of interest, or when performing a cardiac medical procedure, a portion of the heart may be the region of interest.

As described further herein, imager 1040 may be positioned in relation to medical instrument 130 such that the medical instrument is at an angle to the ultrasound image plane, thereby enabling the clinician to visualize the spatial relationship of medical instrument 130 with the ultrasound image plane and with objects being imaged. Further, if provided, the EM tracking system may also track the location of imager 1040. In one or more examples, imager 1040 may be placed inside the body of the patient. The EM tracking system may then track the locations of such imager 1040 and the medical instrument 1030 inside the body of the patient. In some examples, the functions of computing device 150 may be performed by guidance workstation 1052 and computing device 1050 may not be present.

The location of medical instrument 1030 within the body of the patient may be tracked during a therapeutic medical procedure. An exemplary technique of tracking the location of medical instrument 1030 includes using imager 1040 to track the location of medical instrument 1030. Another exemplary technique of tracking the location of medical instrument 1030 includes using the EM tracking system, which tracks the location of medical instrument 1030 by tracking sensors attached to or incorporated in medical instrument 1030. Prior to starting the medical procedure, the clinician may verify the accuracy of the tracking system using any suitable technique or techniques. Any suitable medical instrument 1030 may be utilized with the system 1000. Examples of medical instruments or devices include stents, catheters, angioplasty devices, ablation devices, etc.

Computing device 1050 may be communicatively coupled to imager 1040, workstation 1052, display device 1010 and/or server 1060, for example, by wired, optical, or wireless communications. Server 1060 may be a hospital server which may or may not be located in a Catheter laboratory of the hospital (Cath Lab), a cloud-based server, or the like. Server 1060 may be configured to store patient video data, electronic healthcare or medical records or the like. In some examples, computing device 1050 may be an example of workstation 1052.

Computing device 1050 may be configured to receive imaging data from imager 1040 (e.g., generated by sensors 1070). The imaging data may include image and/or video data. System 1000 may track the motion of medical instrument 1030, such as a guide catheter cannulating a target vessel within a patient. Such tracked motion may saved by computing device 1050, guidance workstation 1052, and/or server 1060 and be used to assess operator technique (e.g., successful medical instrument use) for training purposes, as a variable for research, as input into a machine learning model for training the machine learning model, etc. For example, system 1000 may track a motion of an electrohydraulic intravascular lithotripsy device crossing a calcified lesion. Computing device 1050, guidance workstation 1052, and/or server 1060 may use the tracked motion to assess cross-ability (the ability to cross the lesion) of the particular medical instrument in a particular type of lesion. For example, computing device 1050, guidance workstation 1052, and/or server 1060 may execute a computer vision model to determine a type of lesion that is being treated and based on the tracked motion determine whether the medical instrument successfully crossed the particular lesion. In some examples, computing device 1050, guidance workstation 1052, and/or server 1060 may build a database of types of lesions and medical instruments such that computing device 1050, guidance workstation 1052, and/or server 1060 may determine a likelihood that a particular medical instrument may be successful at crossing or treating a particular type of lesion. In some examples, computing device 1050, guidance workstation 1052, and/or server 1060 may train a machine learning model on the motion of the medical instrument, the type of lesion, and the type of medical instrument being used.

In some examples, during a therapeutic medical procedure, computing device 1050, guidance workstation 1052, and/or server 1060 execute the machine learning model to propose one or more treatment strategies, that may include one or more treatment techniques and one or more medical instruments. For example, computing device 1050, guidance workstation 1052, and/or server 1060 executing the machine learning model may output for display the most likely to be successful treatment strategies for the particular type of lesion.

In some examples, computing device 1050, guidance workstation 1052, and/or server 1060 may save the motion information, the type of lesion, and the type of medical instrument for future viewing by a clinician to facilitate the clinician assessing the particular treatment strategy for the particular lesion type.

Such techniques may be useful as there are several different lesion types, such as bifurcation lesions, calcified lesions, chronic total occlusions (CTOs), in-stent restenosis (ISR), left main disease, etc. There are also many different lesion sub-types (e.g., types within types). For example, the Medina classification system includes seven different sub-types of bifurcation lesions. Moreover, there are multiple treatment techniques for different types of lesions. For example, there are at least six techniques for treating a bifurcation lesion and these techniques may include the use of different medical instruments and/or the use of a different order of the medical instrument(s). As such, the number of different permutations of treatment strategies for a given lesion may be quite large.

Computing device 1050, guidance workstation 1052, and/or server 1060 may execute a computer vision model using the imaging data to determine characteristics of the lesion. For example, computing device 1050, guidance workstation 1052, and/or server 1060 may determine the type of lesion, the sub-type of lesion, or otherwise classify the lesion (e.g., provide a score or other identifier), or the like, based on the determined characteristics of the lesion. Characteristics of the lesion may include, for example, the lesion type (e.g., bifurcation lesion), lesion diameter, the degree of stenosis, the degree of calcification, vessel take-off angles, etc. For example, the Computing device 1050, guidance workstation 1052, and/or server 1060 may execute a machine learning model using the classification of the lesion to determine one or more treatment strategies that are most likely to be successful. The classification of the lesion may be such that lesions having a large degree of similarity are classified the same or close to each other, which in some examples may be called "propensity matching." In this manner, if a lesion has particular characteristics, computing device 1050, guidance workstation 1052, and/or server 1060 may determine one or more treatment strategies that are most likely to be successful for treating a lesion that has those (or nearly those) characteristics and output for display to a clinician the one or more treatment strategies. By providing the one or more treatment strategies that have a highest likelihood of success to a clinician, for example, during a therapeutic medical procedure, the techniques of this disclosure may effect a particular treatment or prophylaxis for a disease or medical condition. These techniques may improve patient outcomes, reduce the need for repeating the therapeutic medical procedure, speed up the therapeutic medical procedure, reduce the exposure of the patient to radioactive contrasts, and preserve medical resources.

In some examples, the classifications of the lesions may be used as a building block that supports clinical research, case planning and/or case analysis (e.g., post therapeutic medical procedure) for those clinicians who want to understand why some techniques work in some cases, but not in other cases. The classifications of the lesions may also be used to highlight the key factors differentiating treatment strategies of two different classifications of lesions to aid in such clinician analysis. In some examples, the classification of the lesions may be used in clinical trials. For example, a number of people having lesions having the same (or very similar) characteristics may be treated with different treatment strategies and the outcomes may be compared.

In some examples, computing device 1050, guidance workstation 1052, and/or server 1060 may execute the computer vision model to determine the medical instrument(s) being used. Alternatively, or additionally, a clinician may enter the type of medical instrument(s) being used via a user interface to computing device 1050, guidance workstation 1052, and/or server 1060. Computing device 1050, guidance workstation 1052, and/or server 1060 may execute computer vision model to determine the success of treatment of the lesion, for example, via the imaging data from the therapeutic medical procedure after the lesion has been treated. In some examples, imaging data post therapeutic medical procedure may be collected and computing device 1050, guidance workstation 1052, and/or server 1060 executing the computer vison model may determine the success of the treatment of the lesion. For example, the success of the treatment of the lesion may include how open the area is after treatment.

In some examples, computing device 1050, guidance workstation 1052, and/or server 1060 may execute a machine learning model to evaluate medical instrument performance and provide guidance on new medical instrument specifications to assist manufacturers to improve the design of such medical instruments for achieving a higher level of success when treating lesions.

Energy generation device 1054, which may be an RDN generator, may be configured to generate energy for an ablation catheter (e.g., medical instrument 1030) which may ablate lesions within a vasculature or heart of a patient. Energy generation device 1054 may also generate information, such as the amount of energy used during an ablation, the length of time of the ablation, error codes, etc. In some examples, energy generation device 1054 may be communicatively coupled to computing device 1050 and/or guidance workstation 1052 and computing device 1050 and/or guidance workstation 1052 may integrate generator data with imaging data. For example, system 1000 may introduce timestamps into the generator data and the imaging data. The timestamps may be used to register the generator data with the imaging data. For example, computing device 1050 and/or guidance workstation 1052 may use the received generator data to determine the location and size of ablated tissue. In some examples, computing device 1050 and/or guidance workstation may use a machine learning model to determine the location and size of the ablated tissue. In some examples, the machine learning model may be resident on server 1060 rather than computing device 1050 or guidance workstation 1052 and computing device 1050 and/or guidance workstation 1052 may communicate with server 1060 to use the machine learning model to determine the location and size of the ablated tissue. Computing device 1050 and/or guidance workstation 1052 may output for display the imaging data together with a representation of the ablated tissue. The representation of the ablated tissue may be overlaid onto the imaging data. In some examples, computing device 1050 and/or guidance workstation 1052 may generate the representation of the ablated tissue using a different color, different shading, different pattern fill, etc. so that the ablated tissue may easily be identified by a clinician on a display, such as display device 1010. In this manner, a clinician may assess the likely success of the ablation period, and determine if further ablation of the particular lesion is desired or if the clinician may move on from the lesion.

If the generator data contains an error code, computing device 1050 and/or guidance workstation 1052 may output for display that an error occurred and the tissue was not ablated as intended. In such a case, computing device 1050, guidance workstation 1052, and/or server 1060 may execute the computer vision model to determine which, if any, tissue was actually ablated. Computing device 1050 and/or guidance workstation 1052 may then output for display the imaging data with a representation of the tissue that was actually ablated. In some examples, when the generator data contains an error code, computing device 1050 and/or guidance workstation 1052 may control energy generation device 1054 to shut energy generation device 1054 down for patient safety reasons until the error code can be examined by the clinician or another person. Computing device 1050 and/or guidance workstation 1052 may also output for display a path through the vasculature of the patient to guide the clinician as they turn the ablation catheter for a next round of ablation(s). Such a path may be represented using a different color, different shading, or different crosshatching so that the path may be easily seen by the clinician.

In some examples, computing device 1050, guidance workstation 1052, and/or server 1060 may store the imaging data having the representation of the ablation for future viewing and/or training of the machine learning model. For example, the stored imaging data having the representation of the ablation may be used to enable more detailed research into patient outcomes and/or for technique and/or medical instrument assessment.

In some examples, system 1000 may include an automated contrast delivery device 1090. In such examples, system 1000 may monitor a time the patient has been subject to the contrast and/or the amount of contrast provided to the patient by automated contrast delivery device. Computing device 1050, guidance workstation 1052, and/or automated contrast delivery device may control automated contrast delivery device 1090 to stop delivering contrast when at least one of the time the patient has been subject to the contrast or the amount of contrast provided to the patient meets a threshold.

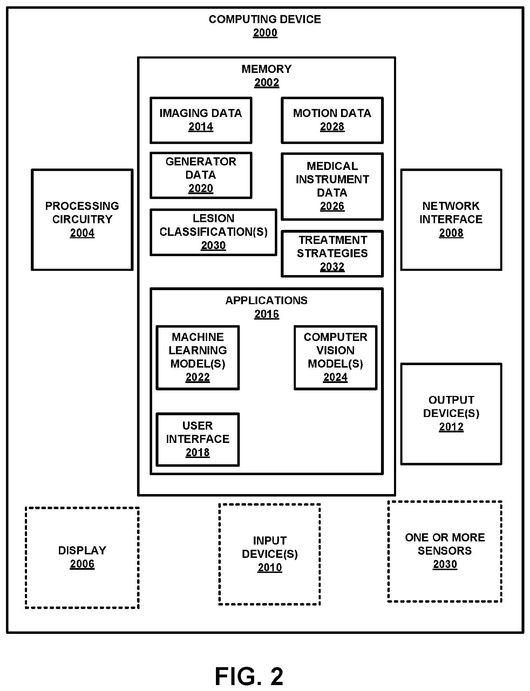

FIG. 2 is a block diagram of one example of a computing device in accordance with one or more aspects of this disclosure. Computing device 2000 may be an example of computing device 1050, guidance workstation 1052, and/or server 1060 of FIG. 1 and may include a workstation, a desktop computer, a laptop computer, a server, a smart phone, a tablet, a dedicated computing device, or any other computing device capable of performing the techniques of this disclosure.

In some examples, computing device 2000 may be configured to perform processing, control and other functions associated with guidance workstation 1052, imager 1040, and an optional EM tracking system. As shown in FIG. 2, computing device 2000 represents multiple instances of computing devices, each of which may be associated with one or more of guidance workstation 1052, imager 1040, or the EM tracking system. Computing device 2000 may include, for example, a memory 2002, processing circuitry 2004, a display 2006, a network interface 2008, an input device(s) 2010, or an output device(s) 2012, each of which may represent any of multiple instances of such a device within the computing system, for ease of description.

While processing circuitry 2004 appears in computing device 2000 in FIG. 2, in some examples, features attributed to processing circuitry 2004 may be performed by processing circuitry of any of computing device 1050, guidance workstation 1052, imager 1040, server 1060, or the EM tracking system, or combinations thereof. In some examples, one or more processors associated with processing circuitry 2004 in computing device 2000 may be distributed and shared across any combination of computing device 1050, guidance workstation 1052, imager 1040, server 1060, and the EM tracking system. Additionally, in some examples, processing operations or other operations performed by processing circuitry 2004 may be performed by one or more processors residing remotely, such as one or more cloud servers or processors, each of which may be considered a part of computing device 2000. Computing device 2000 may be used to perform any of the techniques described in this disclosure, and may form all or part of devices or systems configured to perform such techniques, alone or in conjunction with other components, such as components of computing device 1050, guidance workstation 1052, imager 1040, server 1060, an EM tracking system, or a system including any or all of such devices.

Memory 2002 of computing device 2000 includes any non-transitory computer-readable storage media for storing data or software that is executable by processing circuitry 2004 and that controls the operation of computing device 1050, guidance workstation 1052, imager 1040, server 1060, or EM tracking system, as applicable. In one or more examples, memory 2002 may include one or more solid-state storage devices such as flash memory chips. In one or more examples, memory 2002 may include one or more mass storage devices connected to the processing circuitry 2004 through a mass storage controller (not shown) and a communications bus (not shown).

Although the description of computer-readable media herein refers to a solid-state storage, it should be appreciated by those skilled in the art that computer-readable storage media may be any available media that may be accessed by the processing circuitry 2004. That is, computer readable storage media includes non-transitory, volatile and non-volatile, removable and non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium that may be used to store the desired information and that may be accessed by computing device 2000. In one or more examples, computer-readable storage media may be stored in the cloud or remote storage and accessed using any suitable technique or techniques through at least one of a wired or wireless connection.

Memory 2002 may store machine learning model(s) 2022 and/or computer vision model(s) 2024. In some examples, machine learning model(s) 2022 and computer vision model(s) 2024 may be the same. In other examples, machine learning model(s) 2022 and computer vision model(s) 2024 may be different.

Memory 2002 may store imaging data 2014 and controller data 2020. Imaging data 2014 may be captured by imager 1040 (FIG. 1) during a medical procedure of a patient. Processing circuitry 2004 may receive imaging data 2014 from imager 1040 and store imaging data 2014 in memory 2002. Controller data 2020may be generated by energy generation device 1054 and processing circuitry 2004 may receive controller data 2020from energy generation device 1054. Processing circuitry 2004 may determine a lesion length and/or location based on controller data 2020and generate a representation of the ablated tissue. Processing circuitry 2004 may register controller data 2020and imaging data 2014 using timestamps (which may be placed in the data by, for example, energy generation device 1054, imager 1040, computing device 1050, or guidance workstation 1052). Processing circuitry 2004 may output for display, e.g., to display 2006 or display device 1010, imaging data 2014 with a representation of the ablated tissue overlayed on imaging data 2014. For example, processing circuitry 2004 may execute computer vision model(s) 2024 to recognize anatomical structures, such as vessels, bifurcations, ostia, etc. and may use such recognized anatomical structures to register the imaging data and the representation of the ablated tissue by correlating the two in an x-y plane.

Memory 2002 may also store motion data 2028, medical instrument data 2026, and lesion classification(s) 2030. Motion data 2028 may include tracked motion of medical instrument 1030 through the patient during the therapeutic medical procedure. Motion data 2028 may be generated by EM field generator 1021, by processing circuitry 2004 executing computer vision model(s) 2024 to track the motion of medical instrument 1030 during the therapeutic medical procedure using imaging data from imager 1040, by imager 1040, or by another technique. Motion data 2028 may be indicative of the treatment technique(s) being used, the performance of the clinician using the treatment technique(s), the success of medical instrument 1030 being used with the technique(s) (e.g., the cross-ability of the medical instrument for a particular lesion type or classification). Medical instrument data 2026 may be generated by processing circuitry 2004 executing computer vision model(s) 2024. Computer vision model(s) may be trained to recognize a medical instrument based on imaging data 2014. Alternatively, or additionally, medical instrument data may be input into computing device 2000 by a clinician via input device(s) 2010 or user interface 2018. Medical instrument data 2026 may include the make and model of each medical instrument used during the therapeutic medical procedure. Medical instrument data 2026 may also include other identifying information or information which may be unique to the medical instrument such as a bar code, a year of manufacture, a number of uses, etc.

Memory 2002 may also store treatment strategies 2032. Processing circuitry 2004 executing machine learning model(s) 2022 may determine treatment strategies for presentation to a clinician, for example, during the therapeutic medical procedure. Treatment strategies 2032 may include one or more treatment techniques and one or more medical instruments for use in the therapeutic medical procedure. Generally, treatment strategies 2032 may include one or more of use of a diagnostic catheter, plain old balloon angioplasty (POBA), mechanical atherectomy, intravascular lithotripsy (IVL), drug coated balloon angioplasty, stent delivery (including bare metal stents, drug eluting stents (DES), bioresorbable scaffolds, etc.), post-stenting optimization, wire-based fractional flow reserve (FFR) or other flow reserve measure, image-based FFR or other flow reserve measure, OCT, IVUS, etc. In some examples, treatment strategies 2032 may also include a location of the one or more medical instruments during the therapeutic medical procedure and/or an order of use of the one or more medical instruments. Treatment strategies 2032 may include treatment strategies that are more likely to be successful based on past therapeutic medical procedures.

For example, machine learning model(s) 2022 may be trained using data collected from past therapeutic medical procedures, such as imaging data, tracked motion of medical instruments, generator data, lesion classification or the like. Thus, machine learning model(s) 2022 may be trained on actual treatments and actual outcomes from past therapeutic medical procedures and may include treatment strategies in treatment strategies 2032 based on the treatment strategies that are more likely to result in successful outcomes.

For example, a k-means clustering model may be used having a plurality of clusters: one for each particular treatment technique using one or more particular medical instruments. Each identified lesion may associated with a vector that includes variables for, e.g., type of coronary issue, severity of the coronary issue, complexity of the coronary issue, location of the coronary issue, classification of a lesion, anatomy in the area of the coronary issue, other anatomy, comorbidities of the patient, cholesterol level, blood pressure, blood oxygenation, age, physical exercise level, and/or the like. The location of the vector in a given one of the clusters may be indicative of a particular treatment using one or more particular medical instruments. For example, if the vector falls within the cluster for antegrade dissection re-entry (ADR) using a particular medical instrument, machine learning model(s) 2022 may include ADR as a treatment technique in treatment strategies 2032 and may include the particular medical instrument in treatment strategies 2032.

Alternatively, the k-means clustering algorithm may have a plurality of clusters, one for each classification of a lesion. Each treatment strategy may be associated with a vector that includes variables for, e.g., type of coronary issue, severity of the coronary issue, complexity of the coronary issue, location of the coronary issue, anatomy in the area of the coronary issue, other anatomy, comorbidities of the patient, cholesterol level, blood pressure, blood oxygenation, age, physical exercise level, and/or the like.

Other potential machine learning or artificial intelligence techniques that may be used include Naïve Bayes, k-nearest neighbors, random forest, support vector machines, neural networks, linear regression, logistic regression, etc.

Lesion classification(s) 2030 may be determined by processing circuitry 2004 executing computer vision model(s). For example, computer vision model(s) 2024 may be trained to recognize characteristics of lesions and classify the lesions based on their characteristics, which lesions having the same characteristics being classified the same. For example, computer vision model(s) 2024 may include a convolutional neural network (CNN) which may extract characteristics or features of a lesion to form a vector based on the extracted characteristics. Such a vector may be used to classify the lesion based on other lesions on which computer vision model(s) 2024 was trained. While the use of a CNN is described, other computer vision models may be used.

Processing circuitry 2004 may execute user interface 2018 so as to cause display 2006 (and/or display device 1010 of FIG. 1) to present user interface 2018 to one or more clinicians performing the therapeutic medical procedure. In some examples, user interface 2018 may display, e.g., on display 2006, imaging data 2014 with a representation of an ablated lesion superimposed or overlayed thereon. Memory 2002 may also store machine learning model(s) 2022, computer vision module(s) 2024, and user interface 2018.

Processing circuitry 2004 may be implemented by one or more processors, which may include any number of fixed-function circuits, programmable circuits, or a combination thereof. In various examples, control of any function by processing circuitry 2004 may be implemented directly or in conjunction with any suitable electronic circuitry appropriate for the specified function. Fixed-function circuits refer to circuits that provide particular functionality and are preset on the operations that may be performed. Programmable circuits refer to circuits that may programmed to perform various tasks and provide flexible functionality in the operations that may be performed. For instance, programmable circuits may execute software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. In some examples, the one or more of the units may be distinct circuit blocks (fixed-function or programmable), and in some examples, the one or more units may be integrated circuits.

Instructions may be executed by one or more processors, such as one or more digital signal processors (DSPs), general purpose microprocessors, application specific integrated circuits (ASICs), field programmable gate arrays (FPGAs), graphics processing units (GPUs) or other equivalent integrated or discrete logic circuitry. Accordingly, the term processing circuitry 2004 as used herein may refer to one or more processors having any of the foregoing processor or processing structure or any other structure suitable for implementation of the techniques described herein. In addition, in some aspects, the functionality described herein may be provided within dedicated hardware or software modules configured for encoding and decoding, or incorporated in a combined codec. Also, the techniques could be fully implemented in one or more circuits or logic elements.

Display 2006 may be touch sensitive or voice activated, enabling display 2006 to serve as both an input and output device. Alternatively, a keyboard (not shown), mouse (not shown), or other data input device(s)s (e.g., input device(s) 2010) may be employed.

Network interface 2008 may be adapted to connect to a network such as a local area network (LAN) that includes a wired network or a wireless network, a wide area network (WAN), a wireless mobile network, a Bluetooth network, or the internet. In some examples, network interface 2008 may include one or more application programming interfaces (APIs) for facilitating communication with other devices. For example, computing device 2000 may receive imaging data 2014 from imager 1040 during a therapeutic medical procedure via network interface 2008. Computing device 2000 may also receive controller data 2020 from energy generation device 1054 via network interface 2008. In some examples, computing device 2000 may receive motion data 2028 from, for example, EM field generator 1021 via network interface 2008. Computing device 2000 may receive updates to its software, for example, applications 2016, via network interface 2008. Computing device 2000 may also display notifications on display 2006 that a software update is available.

Input device(s) 2010 may be any device that enables a user to interact with computing device 2000, such as, for example, a mouse, keyboard, foot pedal, touch screen, augmented-reality input device(s) receiving inputs such as hand gestures or body movements, or voice interface.

Output device(s) 2012 may include any connectivity port or bus, such as, for example, parallel ports, serial ports, universal serial busses (USB), or any other similar connectivity port known to those skilled in the art.

Applications 2016 may be one or more software programs stored in memory 2002 and executed by processing circuitry 2004 of computing device 2000. Processing circuitry 2004 may execute user interface 2018, which may display imaging data 2014, a representation of ablated tissue, lesion classifications 2030, and/or treatment strategies 2032 on display 2006 and/or display device 1010 (FIG. 1). Imaging data 2014 and/or the representation of ablated tissue may be stored for future use, such as training and/or performance review of clinicians performing the therapeutic medical procedure. In some examples, processing circuitry 2004 may communicate with server 1060 (FIG. 1) to upload imaging data 2014 during or after the therapeutic medical procedure.

In some examples, processing circuitry 2004 may provide real-time clinical guidance to a clinician. For example, processing circuitry 2004 may use or execute computer visions model(s) 2024 to determine characteristics of a lesion and/or determine a location of a lesion and execute machine learning model(s) 2022 to provide the clinician with proposed treatment strategies.

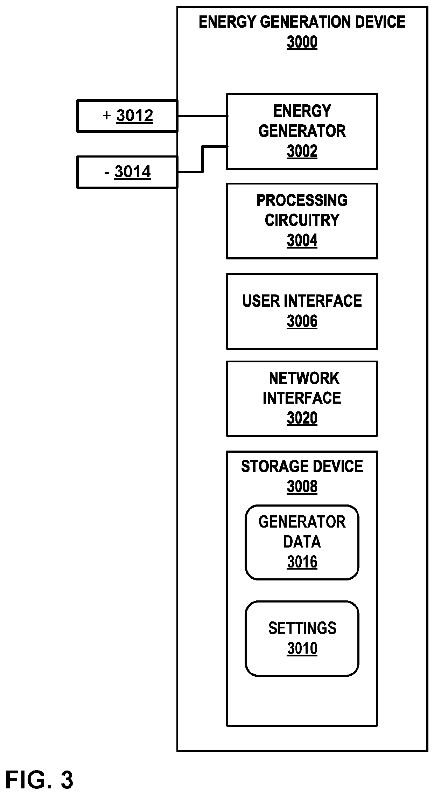

FIG. 3 is a block diagram of an example energy generation device in accordance with one or more aspects of this disclosure. Energy generation device 3000 of FIG. 3 may be an example of energy generation device 1054 (FIG. 1). As shown in FIG. 2, energy generation device 3000 may include positive terminal (+) 3012, negative terminal (-) 3014, energy generator 3002, processing circuitry 3004, user interface 3006, storage device 3008, and network interface 3020.

Positive terminal 3012 may be coupled to energy generator 3002 and may be configured to attach to one or more conductors of an ablation catheter (not shown) so as to conduct electricity between energy generator 3002 and the one or more conductors. Negative terminal 3014 may be coupled to energy generator 3002 (or alternatively to ground) and may be configured to attach to one or more conductors of the ablation catheter so as to conduct electricity between the one or more conductors and energy generator 3002. Energy generator 3002 may be configured to provide radiofrequency electrical pulses to the one or more conductors of the ablation catheter to perform an electroporation procedure or other ablation procedure to lesions such as vascular lesions, cardiac lesions, or other tissues within the patient's body, such as renal tissue, airway tissue, and organs or tissue within the cardiac space or the pericardial space. While shown in the example of FIG. 3 as a single energy generator, energy generation device 3000 is not so limited. For instance, energy generation device 3000 may include multiple energy generators that are each capable of generating ablation signals in parallel. In some examples, energy generation device 3000 may include energy generators of different types, such as a radiofrequency energy generator (such as an RDN generator), a pulsed field energy generator, and/or a cryogenic energy generator.

Processing circuitry 3004 may include one or more processors, such as any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 3004 herein may be embodied as firmware, hardware, software or any combination thereof. Processing circuitry 3004 controls energy generator 3002 to generate signals according to various settings 3010 which may be stored in storage device 3008.

Storage device 3008 may be configured to store controller data 3016 within energy generation device 3000, respectively, during operation. Controller data 3016 may be an example of controller data 2020 and may include an amount of energy delivered during an ablation, an amount of time the energy was delivered, error codes, etc. In some examples, generator data may include timestamps. Storage device 3008 may include a computer-readable storage medium or computer-readable storage device. In some examples, storage device 3008 includes one or more of a short-term memory or a long-term memory. Storage device 3008 may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, storage device 3008 is used to store data indicative of instructions, e.g., for execution by processing circuitry 3004, respectively.

User interface 3006 may include a button or keypad, lights, a speaker/microphone for voice commands, and/or a display, such as a liquid crystal (LCD), light-emitting diode (LED), or organic light-emitting diode (OLED). User interface 3006 may be configured to receive input from a clinician, such as selecting settings from settings 3010 for use during an ablation therapy session. In some examples, the display may be configured to display information regarding an in-progress ablation therapy session, such as patient parameters or other information which may be useful to a clinician.

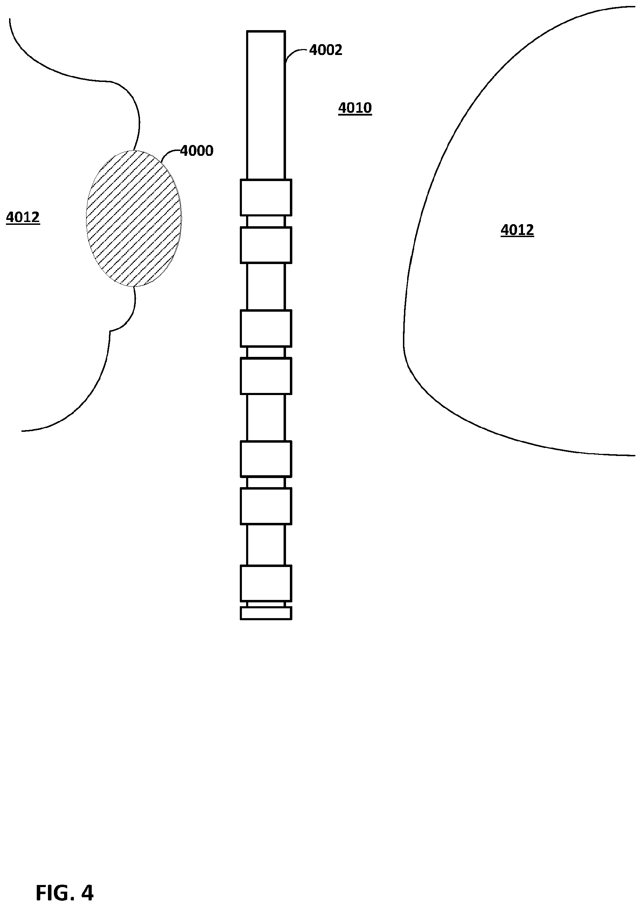

FIG. 4 is a conceptual diagram illustrating the overlaying of a representation of ablated tissue over imaging data in accordance with one or more aspects of this disclosure. FIG. 4 may be an example of information displayed by user interface 2018 (FIG. 2) on display 2006 and/or display device 1010 (FIG. 1) during a therapeutic medical procedure. For example, processing circuitry 2004 (FIG. 2) may generate and output for display a representation of an ablation catheter 4002 (which in some examples may be contained within imaging data 2014 (FIG. 2)) and a representation of ablated tissue 4000, which processing circuitry 2004 may overlay on imaging data 4010 (which may be an example of imaging data 2014). Imaging data 4010 may include tissue 4012 surrounding ablation catheter 4002. In some examples, processing circuitry 2004 may represent the ablated tissue 4000 in a different manner than imaging data 4010, for example, with a different color, different shading, different pattern fill, etc. so that the ablated tissue 4000 may easily be identified by a clinician on a display, such as display 2006 (FIG. 2) or display device 1010 (FIG. 1).

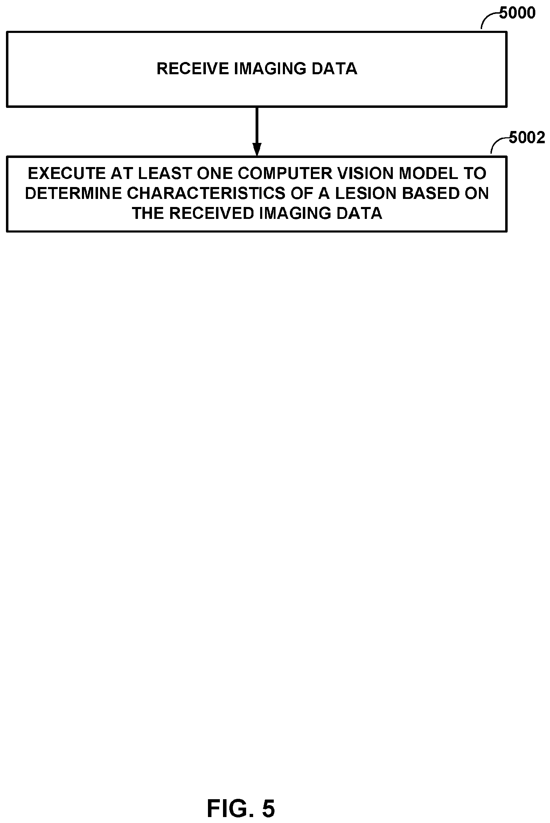

FIG. 5 is a flow diagram illustrating example techniques for determining characteristics of a lesion according to one or more aspects of this disclosure. While described herein with respect to computing device 2000 of FIG. 2, the techniques of FIG. 5 may be implemented by any device of system 1000 (FIG. 1) or any combination of devices of system 1000 capable of performing such techniques. Processing circuitry 2004 may receive imaging data of at least a portion of a vasculature of a patient generated during a cardiac catheterization procedure (5000). For example, processing circuitry 2004 may receive imaging data 2014 of at least a portion of a vasculature of a patient generated during a diagnostic medical procedure or a therapeutic medical procedure from imager 1040 (FIG. 1). Processing circuitry 2004 may execute at least one computer vision model to determine characteristics of a lesion in the vasculature based on the received imaging data (5002). For example, processing circuitry 2004 may execute computer vision model(s) 2024 to determine characteristics of a lesion that appears in imaging data 2014. In some examples, processing circuitry 2004 may execute the at least one computer vision model to determine a degree of success of an ablation of the lesion, for example, during a therapeutic medical procedure. For example, processing circuitry 2004 may execute computer vision model(s) 2024 to examine tissue around the ablation and determine a degree of success of the ablation.

In some examples, the computer vision model is trained on a plurality of lesions in past imaging data of a plurality of patients. In some examples, processing circuitry 2004 may execute the at least one computer vision learning model to determine a medical instrument type of a medical instrument used during the cardiac catheterization procedure. For example, processing circuitry 2004 may execute computer vision model(s) 2024 to determine a make, model, and/or other information of medical instrument 1030 (FIG. 1) or any other medical instrument that is used during the cardiac catheterization procedure. In some examples, the at least one computer vision model is trained on post ablation information in past imaging data from a plurality of patients and processing circuitry 2004 may execute computer vision model(s) 2024 to determine a degree of success of an ablation of the lesion.

In some examples, processing circuitry 2004 may track motion of a medical instrument during the cardiac catheterization procedure and output for display a representation of the motion of the medical instrument during the cardiac catheterization procedure based on the tracked motion and the imaging data. For example, processing circuitry 2004 may receive motion data 2028 (e.g., from EM field generator 1021 (FIG. 1)) or generate motion data 2028 by executing computer vision model 2024 on imaging data 2014 to track the motion of medical instrument 1030. Processing circuitry 2004 may output for display a representation of the motion of the medical instrument (e.g., see representation of medical instrument 4002 in FIG. 4) based on motion data 2028 and imaging data 2014. In some examples, processing circuitry 2004 may determine whether at least a portion of the cardiac catheterization procedure is successful based on the tracked motion.

In some examples, processing circuitry 2004 may execute machine learning model(s) 2022 to guide a clinician during the cardiac catheterization procedure. For example, processing circuitry 2004 may output to display 2006 a representation of a path for the clinician to follow, one or more techniques to employ, one or more medical instruments to use, an order of medical instruments to use, etc. In some examples, processing circuitry 2004 outputs guidance to the clinician during the cardiac catheterization procedure based on the characteristics of the lesion. In some examples, processing circuitry 2004 outputs guidance to the clinician during the cardiac catheterization procedure based on an identity of the clinician. In some examples, processing circuitry 2004 predicts an ability of a medical instrument to cross the lesion based at least in part on the characteristics of the lesion. For example, processing circuitry 2004 may executer machine learning model(s) 2022 to predict the ability of the medical instrument (e.g., medical instrument 1030) to cross the lesion. In some examples, processing circuitry 2004 determines whether a medical instrument crossed a lesion. In some examples, the at least one machine learning model is trained on data collected from past therapeutic medical procedures including at least one of past imaging data, past tracked motion of medical instruments, past controller data, or past lesion classification.

In some examples, processing circuitry 2004 may receive controller data 2020 from a device (e.g., energy generation device 1054 (FIG. 1)). Processing circuitry 2004 may process controller data 2020 to generate a representation of ablated tissue. Processing circuitry 2004 may output for display imaging data 2014 and the representation of the ablated tissue.

In some examples, processing circuitry 2004, prior to outputting for display imaging data 2014 and the representation of the ablated tissue, apply one or more timestamps to imaging data 2014 and apply one or more timestamps to at least one of controller data 2020 or the representation of the ablated tissue. As part of outputting for display imaging data 2014 and the representation of the ablated tissue, processing circuitry 2004 may register imaging data 2014 using the one or more timestamps applied to imaging data 2014 and the one or more timestamps applied to at least one of the controller data 2020 or the representation of the ablated tissue. Processing circuitry 2004 may overlay the representation of the ablated tissue on the imaging data.

In some examples, processing circuitry 2004 may control energy generation device 1054 to stop delivering energy based on controller data 2020. In some examples, processing circuitry 2004 may control automated contrast delivery device 1090 to modulate a contrast delivery rate of a contrast to a patient based on at least one of a quality of the imaging data or in response to a tolerance of the patient to the contrast.

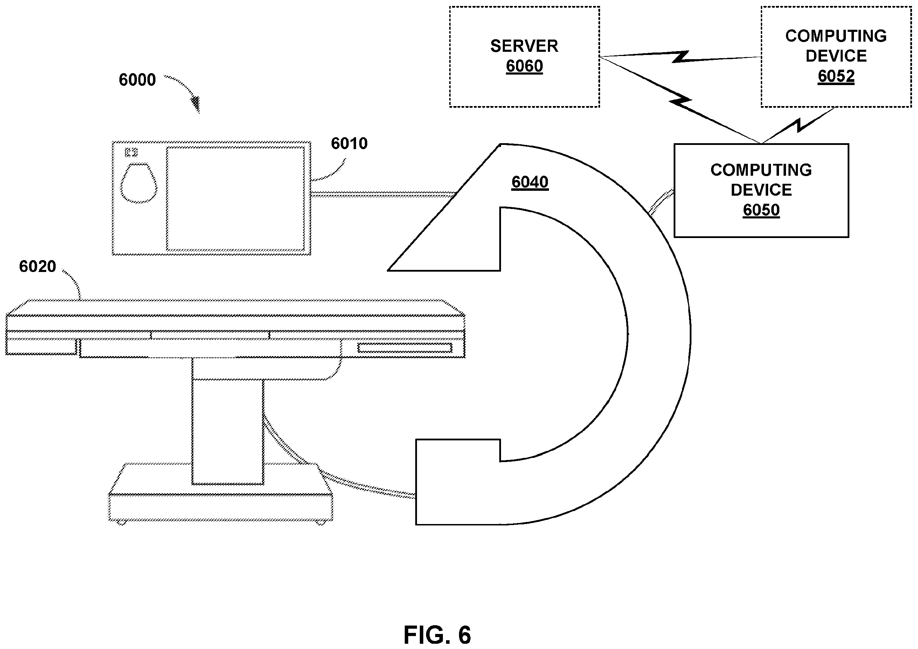

FIG. 6 is a schematic perspective view of one example of a system for determining treatment strategies according to one or more aspects of this disclosure. System 6000 includes a display device 6010, a table 6020, an imager 6040, and a computing device 6050. System 6000 may be an example of a system for use in a Cath lab. In some examples, system 6000 may include other devices, such as additional devices depicted in FIG. 1. Such devices are not shown in FIG. 6 for simplicity purposes. In some examples, system 6000 may also include server 6060 and/or computing device 6052 which may be located in the Cath lab or elsewhere. System 6000 may be used during a diagnostic session to diagnose cardiovascular issues for a patient. During such a diagnostic session (e.g., a diagnostic angiogram), there are three possible outcomes. A first possible outcome is that a clinician may determine no intervention is necessary. A second possible outcome is that a clinician may determine that an urgent intervention is necessary and that the clinician can handle the intervention during the same session (e.g., without the patient leaving and coming back another time). The third possible outcome is that treatment is required, but either the clinician is uncomfortable performing the treatment or that the hospital in which the Cath lab is located does not have the necessary equipment to perform the treatment. In the case of the third possible outcome, imaging data (e.g., angiogram data) from the diagnostic medical procedure may exist which may be used to plan a treatment for the patient. For example, a system may use such imaging data to plan or assist a clinician to plan the treatment.

Such a system may include a computer vision model and a machine learning model. The computer vision model may be used to identify, classify, and/or score a particular lesion. The machine learning model may be used to determine potential treatments having the greatest chances at successful outcomes and may present such potential treatment strategies to a clinician to plan treatment for a therapeutic medical procedure. The system may be configured to run simulations on potential treatment strategies to assist the clinician in selecting one or more treatment strategies to use during the therapeutic medical procedure.

Computing device 6050 may include, for example, an off-the-shelf device such as a laptop computer, desktop computer, tablet computer, smart phone, or other similar device or may include a specific purpose device. Computing device 6050 may perform various control functions with respect to imager 6040. In some examples, computing device 6050 may include a guidance workstation, such as guidance workstation 1052 of FIG. 1. Computing device 6050 may control the operation of imager 6040 and receive the output of imager 6040.

Display device 6010 may be configured to output instructions, images, and messages relating to the diagnostic medical procedure. Table 6020 may be, for example, an operating table or other table suitable for use during a medical procedure such as a diagnostic medical procedure.

In the example of FIG. 6, imager 6040, such as an angiography imager or other imaging device, may be used to image the patient's body during the diagnostic medical procedure to visualize characteristics and locations of lesions inside the patient's body. While described primarily as an angiography imager, imager 6040 may be any type of imaging device, such as an IVUS device, a CT device, an MRI device, a fluoroscopic device, a PET device, an ultrasound device, or the like.

Imager 6040 may image a region of interest in the patient's body. The particular region of interest may be dependent on anatomy, the diagnostic medical procedure, and/or the intended therapy. For example, when performing a diagnostic medical procedure for a cardiovascular issue a portion of the vasculature and/or the heart may be the region of interest.

Computing device 6050 may be communicatively coupled to imager 6040, display device 6010, computing device 6052 and/or server 6060, for example, by wired, optical, or wireless communications. Server 6060 may be a hospital server which may or may not be located in a Catheter laboratory of the hospital (Cath lab), a cloud-based server, or the like. Server 6060 may be configured to store patient imaging data, electronic healthcare or medical records or the like.

Computing device 6052 may include, for example, an off-the-shelf device such as a laptop computer, desktop computer, tablet computer, smart phone, or other similar device or may include a specific purpose device. Computing device 6052 may be a hospital computing device (e.g., owned by the hospital) or may be a personal computing device of a clinician.

Any of, or any combination of, computing device 6050, computing device 6052, and/or server 6060 may include at least one computer vision model and/or at least one machine learning model. For example, computing device 6050, computing device 6052, and/or server 6060 may receive diagnostic imaging data. Computing device 6050, computing device 6052, and/or server 6060 may execute the at least one computer vision model to determine characteristics of a lesion in the received diagnostic imaging data. Computing device 6050, computing device 6052, and/or server 6060 may execute the at least one machine learning model to determine at least one treatment strategy based on the determined characteristic of the lesion, the at least one treatment strategy including at least one treatment technique and at least one medical instrument. In some examples, the at least one treatment strategy may also include information relating to the placement of the at least one medical instrument during the therapeutic medical procedure and/or the order of use of the at least one medical instrument during the therapeutic medical procedure. For example, computing device 6050, computing device 6052, and/or server 6060 may execute the at least one machine learning model to determine one or more treatment strategies having a higher probability of success than other treatment strategies. For example, the at least one machine learning model may predict the probability of success for different treatment strategies based on previous therapeutic medical procedures. For example, the at least one machine learning algorithm may analyze treatment strategies from previous therapeutic medical procedures for lesions having the same or similar characteristics. For example, the machine learning model may determine that there is a 40% chance of crossing the lesion with the electrohydraulic intravascular lithotripsy if there is no pre-dilation, but a 75% chance of crossing the lesion with low profile noncompliant (NC) balloon. Computing device 6050, computing device 6052, and/or server 6060 may output for display to a clinician the at least one treatment technique, for example on display device 6010 or a display of computing device 6050 or computing device 6052.

In some examples, computing device 6050, computing device 6052, and/or server 6060 may be configured to, responsive to input of a clinician, run one or more simulations of using any of the at least one treatment strategy to treat the lesion. In some examples, computing device 6050, computing device 6052, and/or server 6060 may superimpose the simulation(s) or the results of the simulation(s) on the actual imaging data, or a still image from the imaging data showing the lesion, in the display.

In some examples, computing device 6050, computing device 6052, and/or server 6060 may be configured to receive a user input of a selected at least one treatment technique and a selected at least one medical instrument and computing device 6050, computing device 6052, and/or server 6060 may amend the at least one treatment strategy accordingly. For example, a clinician may, after seeing the at least one treatment strategy and possibly after running one or more simulations, may determine that a particular treatment strategy is one that the clinician intends to use and may delete any other proposed treatment strategies for the lesion. Additionally, or alternatively, the clinician may determine that a particular treatment strategy might be one that the clinician may use if one or more of the treatment techniques and/or one or more of medical instruments within the treatment strategy is changed. In such a case, the clinician may change, via a user interface, any of the treatment techniques and/or medical instruments within the treatment strategy. Additionally, or alternatively, the clinician may prepare their own treatment strategy which they may add to the at least one treatment strategy or with which they may replace the at least one treatment strategy. In some examples, computing device 6050, computing device 6052, and/or server 6060 may, responsive to clinician input, run a further simulation using the amended treatment strategy for treating the lesion. These techniques may be iteratively repeated until the clinician is satisfied with the resulting at least one treatment strategy. In some examples, these techniques may be used to train Fellows or may be used by a clinician to plan a therapeutic medical procedure.

Such techniques may be useful as there are several different lesion types, such as bifurcation lesions, calcified lesions, chronic total occlusions (CTOs), in-stent restenosis (ISR), left main disease, etc. There are also many different lesion sub-types (e.g., types within types). For example, the Medina classification system includes seven different sub-types of bifurcation lesions. Moreover, there are multiple treatment techniques for different types of lesions. For example, there are at least six techniques for treating a bifurcation lesion and these techniques may include the use of different medical instruments and/or the use of a different order of the medical instrument(s). As such, the number of different permutations of treatment strategies for a given lesion may be quite large.

By providing the one or more treatment strategies that have a highest likelihood of success to a clinician, for example, for planning a therapeutic medical procedure, the techniques of this disclosure may effect a particular treatment or prophylaxis for a disease or medical condition. These techniques may improve patient outcomes, reduce the need for repeating the therapeutic medical procedure, speed up the therapeutic medical procedure, reduce the exposure of the patient to radioactive contrasts, and/or preserve medical resources.

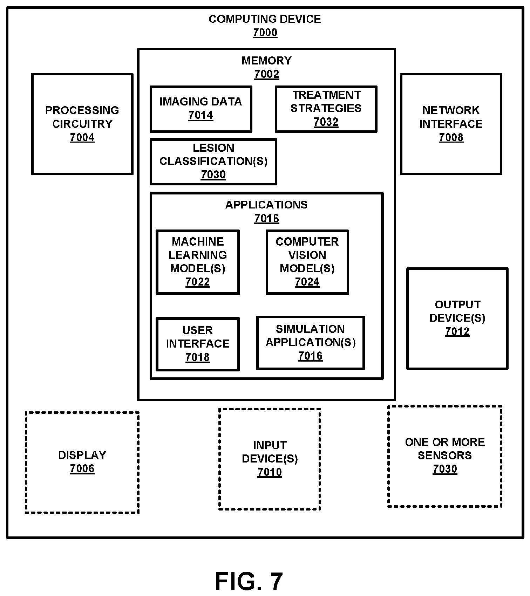

FIG. 7 is a schematic view of one example of a computing device in accordance with one or more aspects of this disclosure. Computing device 7000 may be an example of computing device 6050, computing device 6052, and/or server 6060 of FIG. 6 and may include a workstation, a desktop computer, a laptop computer, a server, a smart phone, a tablet, a dedicated computing device, or any other computing device capable of performing the techniques of this disclosure.

Computing device 7000 may include, for example, a memory 7002, processing circuitry 7004, a display 7006, a network interface 7008, input device(s) 7010, and/or an output device(s) 7012, each of which may represent any of multiple instances of such a device within the computing system, for ease of description.

While processing circuitry 7004 appears in computing device 7000 in FIG. 7, in some examples, features attributed to processing circuitry 7004 may be performed by processing circuitry of any devices of system 6000 (FIG. 6), or combinations thereof. In some examples, one or more processors associated with processing circuitry 7004 in computing device 7000 may be distributed and shared across any combination of computing device 6050, computing device 6052, and 6060. Additionally, in some examples, processing operations or other operations performed by processing circuitry 7004 may be performed by one or more processors residing remotely, such as one or more cloud servers or processors, each of which may be considered a part of computing device 7000. Computing device 7000 may be used to perform any of the techniques described in this disclosure, and may form all or part of devices or systems configured to perform such techniques, alone or in conjunction with other components, such as components of computing device 6050, computing device 6052, server 6060, or a system including any or all of such devices.

Memory 7002 of computing device 7000 includes any non-transitory computer-readable storage media for storing data or software that is executable by processing circuitry 7004 and that controls the operation of computing device 7000. In one or more examples, memory 7002 may include one or more solid-state storage devices such as flash memory chips. In one or more examples, memory 7002 may include one or more mass storage devices connected to the processing circuitry 7004 through a mass storage controller (not shown) and a communications bus (not shown).

Although the description of computer-readable media herein refers to a solid-state storage, it should be appreciated by those skilled in the art that computer-readable storage media may be any available media that may be accessed by the processing circuitry 7004. That is, computer readable storage media includes non-transitory, volatile and non-volatile, removable and non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium that may be used to store the desired information and that may be accessed by computing device 7000. In one or more examples, computer-readable storage media may be stored in the cloud or remote storage and accessed using any suitable technique or techniques through at least one of a wired or wireless connection.

Memory 7002 may store machine learning model(s) 7022 and/or computer vision model(s) 7024. In some examples, machine learning model(s) 7022 and computer vision model(s) 7024 may be the same. In other examples, machine learning model(s) 7022 and computer vision model(s) 7024 may be different.

Memory 7002 may store imaging data 7014. Imaging data 7014 may be captured by imager 6040 (FIG. 6) during a diagnostic medical procedure of a patient. Processing circuitry 7004 may receive imaging data 7014 from imager 6040 and store imaging data 7014 in memory 7002.

Memory 7002 may also store lesion classification(s) 7030 such as a classification of a lesion appearing in imaging data 7014. Processing circuitry 6004 executing computer vision model(s) 7024 may determine a classification of the lesion which may be stored in lesion classification(s). For example, processing circuitry 6004 may classify a lesion based on characteristics of the lesion, as discussed above with respect to FIGS. 1-2. Memory 7002 may also store treatment strategies 7032. Processing circuitry 7004 executing machine learning model 7022 may determine treatment strategies for presentation to a clinician, for example, to assist in planning of a therapeutic medical procedure or for training purposes. Treatment strategies 7032 may include one or more treatment techniques and one or more medical instruments for use in the therapeutic medical procedure. Generally, treatment strategies may 7032 include one or more of use of a diagnostic catheter, plain old balloon angioplasty (POBA), mechanical atherectomy, intravascular lithotripsy (IVL), drug coated balloon angioplasty, stent delivery (including bare metal stents, drug eluting stents (DES), bioresorbable scaffolds, etc.), post-stenting optimization, wire-based FFR or other flow reserve measure, image-based FFR or other flow reserve measure, OCT, IVUS, etc. In some examples, treatment strategies 7032 may also include a location of the one or more medical instruments during the therapeutic medical procedure and/or an order of use of the one or more medical instruments. Treatment strategies 7032 may include treatment strategies that are more likely to be successful based on past therapeutic medical procedures.

For example, machine learning model(s) 7022 may be trained using data collected from past therapeutic medical procedures, such as imaging data, tracked motion of medical instruments, generator data, lesion classification or the like. Thus, machine learning model(s) 7022 may be trained on actual treatments and actual outcomes from past therapeutic medical procedures and may include treatment strategies in treatment strategies 7032 based on the treatment strategies that are more likely to result in successful outcomes.

For example, a k-means clustering model may be used having a plurality of clusters: one for each particular treatment technique using one or more particular medical instruments. Each identified lesion may associated with a vector that includes variables for, e.g., type of coronary issue, severity of the coronary issue, complexity of the coronary issue, location of the coronary issue, classification of a lesion, anatomy in the area of the coronary issue, other anatomy, comorbidities of the patient, cholesterol level, blood pressure, blood oxygenation, age, physical exercise level, and/or the like. The location of the vector in a given one of the clusters may be indicative of a particular treatment using one or more particular medical instruments. For example, if the vector falls within the cluster for antegrade dissection re-entry (ADR) using a particular medical instrument, machine learning model(s) 7022 may include ADR as a treatment technique in treatment strategies 7032 and may include the particular medical instrument in treatment strategies 7032.

Alternatively, the k-means clustering algorithm may have a plurality of clusters, one for each classification of a lesion. Each treatment strategy may be associated with a vector that includes variables for, e.g., type of coronary issue, severity of the coronary issue, complexity of the coronary issue, location of the coronary issue, anatomy in the area of the coronary issue, other anatomy, comorbidities of the patient, cholesterol level, blood pressure, blood oxygenation, age, physical exercise level, and/or the like.

Other potential machine learning or artificial intelligence techniques that may be used include Naïve Bayes, k-nearest neighbors, random forest, support vector machines, neural networks, linear regression, logistic regression, etc.

Lesion classification(s) 7030 may be determined by processing circuitry 7004 executing computer vision model(s). For example, computer vision model(s) 7024 may be trained to recognize characteristics of lesions and classify the lesions based on their characteristics, which lesions having the same (or nearly the same) characteristics being classified the same. For example, computer vision model(s) 7024 may include a convolutional neural network (CNN) which may extract characteristics or features of a lesion to form a vector based on the extracted characteristics. Such a vector may be used to classify the lesion based on other lesions on which computer vision model(s) 7024 was trained. While the use of a CNN is described, other computer vision models may be used.

Processing circuitry 7004 may execute user interface 7018 so as to cause display 7006 (and/or display device 6010 of FIG. 6) to present user interface 7018 to a clinician preparing for a therapeutic medical procedure or a clinician undergoing training. In some examples, user interface 7018 may display, e.g., on display 7006, treatment strategies 7032 which processing circuitry executing machine learning model(s) 7022 may determine have better chances for successful outcomes than other treatment strategies for a particular classification of the lesion of the patient.

In some examples, the clinician may desire to run a simulation on at least one of treatment strategies 7032. In such a case, the clinician may provide user input via user interface 7018 or input device(s) 7010 indicating that processing circuitry 7004 should run such a simulation on a selected treatment strategy of treatment strategies 7032. Processing circuitry 7004 may then load simulation application(s) 7016 from memory and execute simulation application(s) 7016 on the selected treatment strategy.

In some example, the clinician may desire to delete certain treatment strategies from treatment strategies 7032, change certain aspects (e.g., one or more treatment techniques, medical instruments, location of medical instruments, and/or the order of use of the medical instruments) of a given treatment strategy, or to create a treatment strategy not in treatment strategies 7032. In such a case, the clinician may provide user input via user interface 7018 or input device(s) 7010 indicating that the clinician would like to amend treatment strategies 7032 and processing circuitry 7004 may facilitate the clinician amending treatment strategies 7032. Memory 7002 may also store machine learning model(s) 7022, computer vision module(s) 7024, and user interface 7018.

Processing circuitry 7004 may be implemented by one or more processors, which may include any number of fixed-function circuits, programmable circuits, or a combination thereof. In various examples, control of any function by processing circuitry 7004 may be implemented directly or in conjunction with any suitable electronic circuitry appropriate for the specified function. Fixed-function circuits refer to circuits that provide particular functionality and are preset on the operations that may be performed. Programmable circuits refer to circuits that may programmed to perform various tasks and provide flexible functionality in the operations that may be performed. For instance, programmable circuits may execute software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. In some examples, the one or more of the units may be distinct circuit blocks (fixed-function or programmable), and in some examples, the one or more units may be integrated circuits.

Instructions may be executed by one or more processors, such as one or more digital signal processors (DSPs), general purpose microprocessors, application specific integrated circuits (ASICs), field programmable gate arrays (FPGAs), graphics processing units (GPUs) or other equivalent integrated or discrete logic circuitry. Accordingly, the term processing circuitry 7004 as used herein may refer to one or more processors having any of the foregoing processor or processing structure or any other structure suitable for implementation of the techniques described herein. In addition, in some aspects, the functionality described herein may be provided within dedicated hardware or software modules configured for encoding and decoding, or incorporated in a combined codec. Also, the techniques could be fully implemented in one or more circuits or logic elements.

Display 7006 may be touch sensitive or voice activated, enabling display 7006 to serve as both an input and output device. Alternatively, a keyboard (not shown), mouse (not shown), or other data input device(s)s (e.g., input device(s) 7010) may be employed.

Network interface 7008 may be adapted to connect to a network such as a local area network (LAN) that includes a wired network or a wireless network, a wide area network (WAN), a wireless mobile network, a Bluetooth network, or the internet. In some examples, network interface 7008 may include one or more application programming interfaces (APIs) for facilitating communication with other devices. For example, computing device 7000 may receive imaging data 7014 from imager 6040 during or after a diagnostic medical procedure via network interface 7008. Computing device 7000 may receive updates to its software, for example, applications 7016, via network interface 7008. Computing device 7000 may also display notifications on display 2006 that a software update is available.

Input device(s) 7010 may be any device that enables a user to interact with computing device 7000, such as, for example, a mouse, keyboard, foot pedal, touch screen, augmented-reality input device(s) receiving inputs such as hand gestures or body movements, or voice interface.

Output device(s) 7012 may include any connectivity port or bus, such as, for example, parallel ports, serial ports, universal serial busses (USB), or any other similar connectivity port known to those skilled in the art.

Applications 7016 may be one or more software programs stored in memory 7002 and executed by processing circuitry 7004 of computing device 7000. Processing circuitry 7004 may execute user interface 7018, which may display treatment strategies 7032, simulations, lesion classification(s) 7030, and/or imaging data 7014 on display 7006 and/or display device 6010 (FIG. 6).

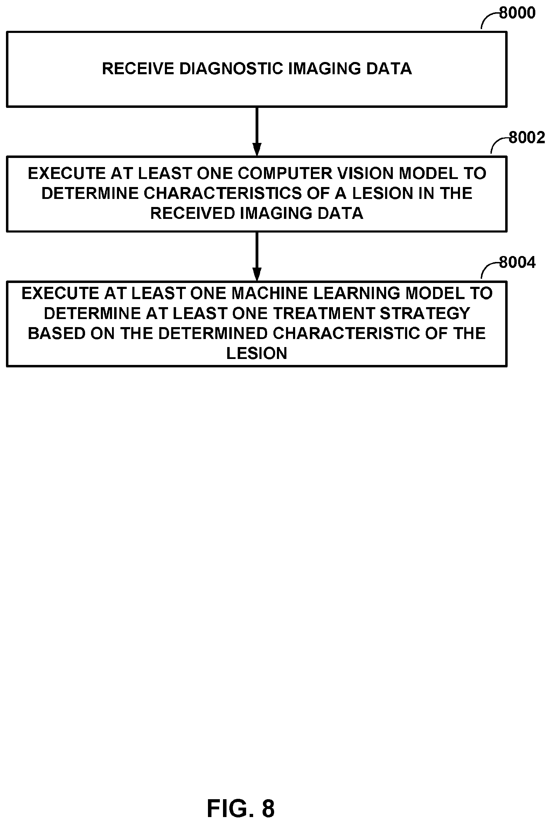

FIG. 8 is a flow diagram illustrating example techniques for determining treatment strategies according to one or more aspects of this disclosure. While described herein with respect to computing device 7000 of FIG. 7, the techniques of FIG. 7 may be implemented by any device of system 6000 (FIG. 6) or any combination of devices of system 6000 capable of performing such techniques. Processing circuitry 7004 may receive diagnostic imaging data of at least a portion of a vasculature of a patient generated during a cardiac diagnostic medical procedure (8000). For example, processing circuitry 7004 may receive imaging data 7014 from imager 6040, computing device 6050, server 6060, computing device 6052, or from memory, such as a thumb drive.

Processing circuitry 7004 may execute at least one computer vision model to determine characteristics of a lesion in the vasculature based on the received diagnostic imaging data (8002). For example, processing circuitry 7004 may execute computer vision model(s) 7024 to determine characteristics of a lesion in imaging data 7014. Processing circuitry 7004 may execute at least one machine learning model to determine at least one treatment strategy based on the determined characteristic of the lesion, the at least one treatment strategy including at least one treatment technique and at least one medical instrument (8004). For example, processing circuitry 7004 may execute machine learning model(s) 7022 to determine treatment strategies 7032 based on lesion classification(s) 7030.

In some examples, processing circuitry 7004 is further configured to output treatment strategies (which may be a single treatment strategy) 7032 for display. In some examples, treatment strategies 7032 further includes an indication of a predicted degree of success of how likely the use of the at least one treatment technique and the at least one medical instrument may be successful. In some examples, processing circuitry 7004 is further configured to, in response to user input, execute a first simulation of a first medical procedure using the at least one treatment strategy. For example, processing circuitry 7004 may load simulation application(s) 7016 and execute simulation application(s) 7016 simulating the use of the treatment strategy selected by the clinician for simulation on the lesion. In some examples, the simulation is based, at least in part, on the received diagnostic imaging data (e.g., imaging data 7014).

In some examples, processing circuitry 7004 may receive user input of a selected at least one treatment technique. Processing circuitry 7004 may be configured to receive user input amending the selected at least one treatment strategy and amend the selected at least one treatment strategy (e.g., treatment strategies 7032) based on the user input to generate at least one amended treatment strategy.

In some examples, processing circuitry 7004 may execute a second simulation of a second medical procedure using the at least one amended treatment strategy. In some examples, the at least one amended treatment strategy comprises at least one of a selected at least one treatment technique and/or at least one medical instrument. In some examples, the at least one treatment strategy does not comprise at least one of the selected at least one treatment technique or the at least one of the selected medical instrument.

In some examples, machine learning model(s) 7022 is trained on imaging data from prior therapeutic medical procedures, a plurality of lesion types, a plurality of medical instruments, a plurality of clinicians, and a plurality of therapeutic medical procedures.

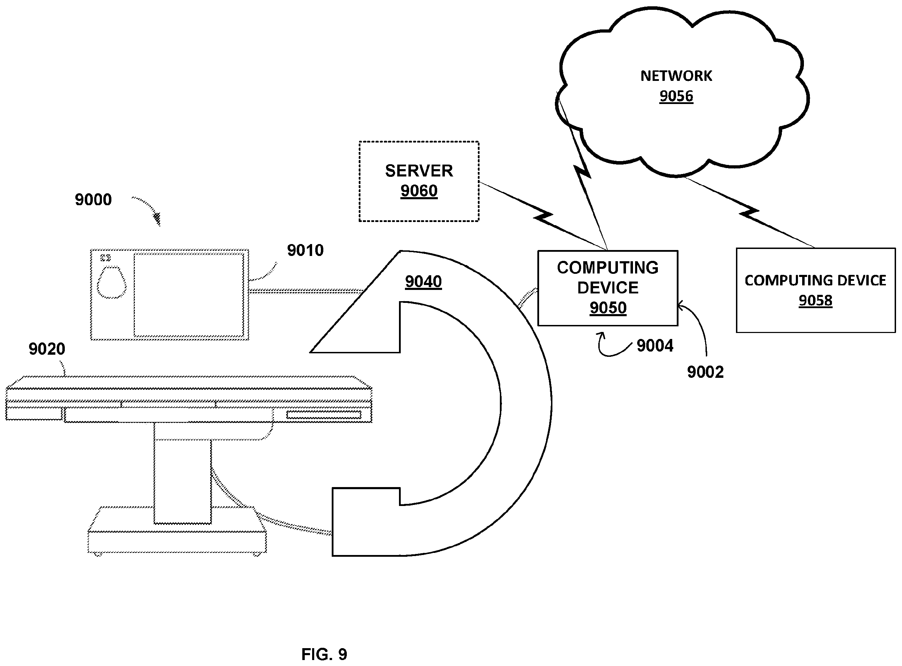

FIG. 9 is a schematic perspective view of example medical system 9000. Medical system 9000 may be an example of medical system 1000 of FIG.1 and/or medical system 6000 of FIG. 6. Medical system 9000 of FIG. 9 is similar to medical system 6000 of FIG. 6, differing as described below, where similar reference numbers indicate similar elements. Medical system 9000 may provide a system for establishing a communication session between an operating clinician and a remote clinician and streaming imaging data representative of a medical procedure to the remote clinician for a consult.

System 9000 includes a display device 9010, a table 9020, an imager 9040, a first computing device 9050, a server 9060, a network 9056, and a second computing device 9058. System 9000 may be an example of a system for use in a Cath lab. In some examples, system 9000 may include other devices, such as additional devices depicted in FIG. 1, which are not shown in FIG. 9 for simplicity purposes. System 9000 may be used during a diagnostic session to diagnose cardiovascular issues for a patient. As discussed above, during such a diagnostic session (e.g., a diagnostic angiogram), there are three possible outcomes. A first possible outcome is that a clinician may determine no intervention is necessary. A second possible outcome is that a clinician may determine that an urgent intervention is necessary and that the clinician can handle the intervention during the same session (e.g., without the patient leaving and coming back another time). The third possible outcome is that treatment is required, but either the clinician is uncomfortable performing the treatment or that the hospital in which the Cath lab is located does not have the necessary equipment to perform the treatment. In the case of the third possible outcome, imaging data (e.g., angiogram data) from the diagnostic medical procedure may exist which may be used to plan a treatment for the patient. System 9000 may move cases from the third possible outcome to the second possible outcome by allowing a consultation from a remote clinician, which may make the operating clinician more comfortable with a procedure, and thus able to provide necessary medical treatment to a patient during the same session.

First computing device 9050 may be associated with a first clinician, who may be located in the Cath Lab during the medical procedure. First computing device 9050 may be an example of computing device 6050 or 6052 (FIG. 6) and may include, for example, an off-the-shelf device, such as a laptop computer, desktop computer, tablet computer, smart phone, or other similar device. First computing device 9050 includes memory 9002 and processing circuitry 9004. Second computing device 9058 may be associated with a second clinician located remotely. For example, second computing device may be associated with a second clinician located in another part of the hospital, at an expert call center, at home, on vacation, or the like. Similarly to first computing device 9050, second computing device 9058 may include, for example, an off-the-shelf device, such as a laptop computer, desktop computer, tablet computer, smart phone, or other similar device.

While processing circuitry 9004 appears in first computing device 9050 in FIG. 9, in some examples, features attributed to processing circuitry 9004 may be performed by processing circuitry of any of first computing device 9050, second computing device 9058, imager 9040, server 9060, network 9056, other elements of system 9000, or combinations thereof. In some examples, one or more processors associated with processing circuitry 9004 in first computing device 9050 may be distributed and shared across any combination of first computing device 9050, second computing device 9058, imager 9040, server 9060, network 9056, and display device 9010. Additionally, in some examples, processing operations or other operations performed by processing circuitry 9004 may be performed by one or more processors residing remotely, such as one or more cloud servers or processors, each of which may be considered a part of computing device 9050.

System 9000 includes network 9056, which is a suitable network such as a local area network (LAN) that includes a wired network or a wireless network, a wide area network (WAN), a wireless mobile network, a Bluetooth network, or the internet. In some examples, network 9056 may be a secure network, such as a hospital network, which may limit access by users.

Processing circuitry 9004 may communicatively couple first computing device 9050, being operated by a clinician or assistant in the Cath Lab, and second computing device 9058 associated with a second clinician located remotely. In some examples, the first clinician may be performing a medical procedure such as a cardiac catheterization lab procedure, and may encounter a patient condition which they are uncomfortable treating (e.g., a lesion or lesions of particular complexity). In such a case, a first clinician may input a request through a user interface at display device 9010, and computing device 9050 may relay the representation of user input to request a consult from a second clinician by, for example, a call or message to second computing device 9058 associated with the second clinician, located elsewhere as discussed above. Responsive to receiving the representation of user input to request the consult, a communication session between first computing device 9050 and second computing device 9058 may be established. During the communication session, system 9000 may be configured to stream a representation of data captured by imager 9040 of the medical procedure. As discussed above, the representation of data captured by imager 9040 may be photographic or video data.

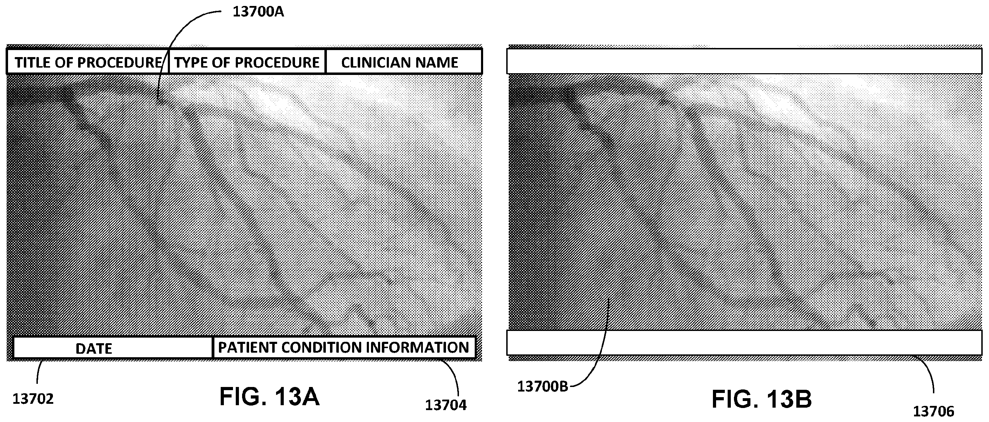

As discussed above, imager 9040 may be an angiography imager or other imaging device, and may be used to image the patient's body during the procedure to visualize characteristics and locations of lesions inside the patient's body. Imager 9040 may be any type of imaging device, such as a CT device, an MRI device, a fluoroscopic device, a PET device, an ultrasound device, or the like. One or more of these imaging devices may capture, as part of its normal operation, personal health information (PHI) from a patient which may not be approved for sharing with certain clinicians who may serve as the second, remote clinician. For example, a first clinician may request a consult from a former attending physician, schoolmate, professor, or the like who is not affiliated with the hospital and thus not approved to view PHI related to the patient. In some examples, processing circuitry 9004 may be configured to determine a permission state of the second clinician and redact, prior to streaming and responsive to determining that the permission state of the second clinician is below a threshold permission state, personal health information from the representation of data captured by the one or more image sensors, as will be described in further detail with respect to FIGS. 13A and 13B below.

In some examples, processing circuitry 9004 may assist the first clinician in determining when and whether to request a consult from a second clinician. For example, processing circuitry 9004 may be configured to execute a computer vision model such as computer vision model 2024 (FIG. 2) and/or machine learning model 2022 (FIG. 2) to recognize a patient condition or an intraprocedural event in the representation of data captured by imager 9040. Responsive to recognizing a patient condition or an intraprocedural event (e.g., a malfunctioning or broken tool or instrument, an approach to a lesion, or the like), processing circuitry 9004 may present, at display device 9010, a user interface including an option to the first clinician to request a consult from second computing device 9058.

Although second computing device 9058 is described above as a single computing device associated with a single second clinician located remotely, in some examples processing circuitry 9004 may be configured to receive a representation of a request by the first clinician to message or call more than one second computing device associated with more than one second clinician. In this way, processing circuitry 9004 may be configured to allow a clinician to reach out to a number of potential consulting clinicians during a medical procedure, and receive a consultation even if one or more than one of the potential second clinicians are not available.

In some examples, processing circuitry 9004 may be configured to receive annotations on the representation of data from one or both of the first clinician through first computing device 9050 or the second clinician through second computing device 9058, and transmit the annotations to the other of the first clinician or the second clinician during the communication session. Such annotation may include notes or markings indicating, for example, a suggested pathway. Additionally, or alternatively, processing circuitry 9004 may be configured to capture data from one or more audio sensors (not pictured) during the medical procedure and stream the data captured from the one or more audio sensors during the communication session. Audio sensors may be off the shelf components of a laptop, tablet, mobile phone, or the like or may be a part of a Cath Lab.

In some examples, processing circuitry 9004 may be configured to cause a display associated with second computing device 9058 a list of medical devices available to the first clinician. Processing circuitry configured to share the list of instruments and tools available to the operating clinician may help the second clinician to deliver the best advice or recommendation possible under the circumstances, since it is considered that the operating clinician may be in a non-state of the art setting and without access to some or all of the best instruments and equipment when performing the medical procedure.

In some examples, processing circuitry 9004 may be configured to link the data from imager 9040 captured during the medical procedure to data from another medical test or procedure. The other medical test or procedure may take place prior to or during the medical procedure that is being streamed via the communication setting. For example, a patient may be undergoing a percutaneous coronary intervention, the streamed medical procedure may be a cardiac catheterization procedure, and the data from another medical test or procedure may be data from a prior calcification test. In some examples, the calcification test may be a Coronary Artery Calcium Score, which may be generated by computed tomography (CT). In some examples, the CT scan may occur before the PCI procedure. In some examples, the other medical test or procedure may include data generated via intravascular ultrasound, which, in some examples, may be generated during the medical procedure In some examples, the other medical test may include data generated during an echocardiogram. Echocardiogram data may be especially important in examples where the medical procedure is a structural heart medical procedure.

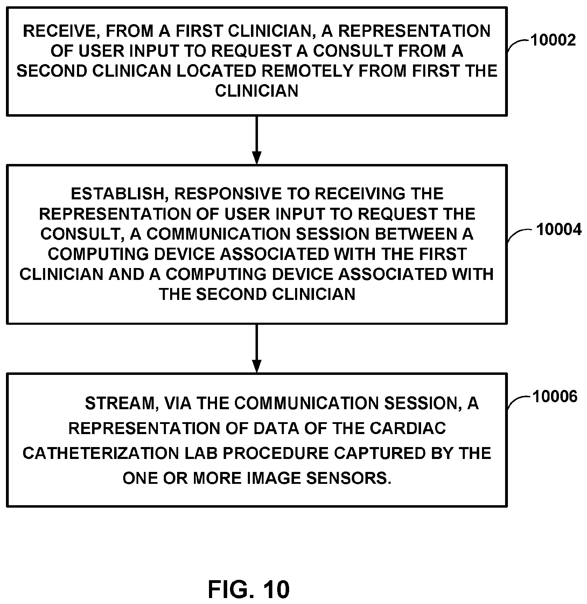

FIG. 10 is a flow diagram illustrating example techniques for streaming a representation of data to a remote clinician according to one or more aspects of this disclosure. While described herein with respect to system 9000 of FIG. 9, the techniques of FIG. 9 may be implemented other systems such as system 1000 (FIG. 1), system 6000 (FIG. 6) or any combination of devices of system 9000 capable of performing such techniques.

Processing circuitry 9004 may receive, from a first clinician that is performing a medical procedure and is associated with first computing device 9050, a representation of user input to request a consult from a second clinician that is located remotely from the first clinician (10002). In some examples, processing circuitry 9004 may execute a computer vision model to recognize a patient condition or an intraprocedural event in the representation of data captured by imager 9040. Further, in some examples, processing circuitry 9004 may cause display device 9010 to display a user interface including an option to the first clinician to request a consult from second computing device 9058 associated with the second clinician. In some examples, processing circuitry 9004 may receive a representation of user input to request a consult from the second clinician, and processing circuitry 9004 may cause first computing device 9050 to call or message second computing device 9058. In some examples, the patient condition may be a lesion, and the medical procedure may be a percutaneous coronary intervention.

Processing circuitry 9004 may establish, responsive to receiving the representation of user input to request the consult, a communication session between a first computing device 9050 and second computing device 9058 (10004).

In some examples, the technique of FIG. 10 may include processing circuitry 9004 determining a permission state of the second clinician. Processing circuitry 90004 may redact, prior to streaming and responsive to determining that the permission state of the second clinician is below a threshold permission state, personal health information from the representation of data captured by imager 9040.

Processing circuitry 9004 may stream, via the communication session, a representation of data of the medical procedure captured by imager 9040 (10006). In some examples, data captured by the imager 9040 may include video data, fluoroscopy imaging, or both. In some examples, processing circuitry 9004 may receive annotations on the representation of data from one or both of the first clinician or the second clinician through their respective associated computing device. In some examples, processing circuitry 9004 may transmit the annotations to the respective computing device of the other of the first clinician or the second clinician during the communication session. In some examples, the stream during the communication session may further include data captured by processing circuitry 9004 from one or more audio sensors during the medical procedure. In some examples, processing circuitry 9004 may cause a display associated with second computing device 9058 to display a list of medical equipment available to the first clinician. In some examples, processing circuitry 9004 may link the data from imager 9040 during the medical procedure to data from another medical test or procedure. In some examples, the other medical test or procedure is a calcification test or data generated during an intravascular ultrasound medical procedure.

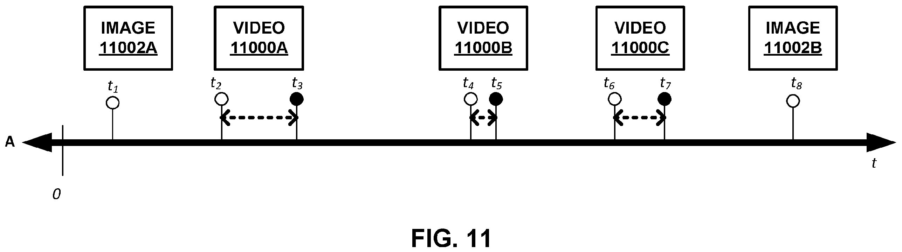

FIG. 11 is a time diagram illustrating example condensed versions of imaging data sensed by one or more image sensors during a cardiac catheterization medical procedure according to one or more aspects of the present disclosure. FIG. 11 illustrates a time dimension along axis A beginning at time 0 and extending to time *t*. Referring concurrently to FIGS. 9 and 11, medical system 9000 may be configured to receive imaging data from imager 9040. The imaging data may be sensed by imager 9004 during a medical procedure such as a cardiac catheterization procedure. Imaging data may be sensed as a substantially continuous stream of discrete images beginning at time 0, which may correspond to a time that a medical procedure starts and/or imager 9040 is turned on. The continuous stream of discrete images beginning at time 0, and extend continuously until time *t.*

Processing circuitry 9004 may be configured to generate a condensed version of the imaging data, the condensed version of the imaging data including images corresponding to particular events of the medical procedure. Stated similarly, processing circuitry 9004 may create a shortened version of the medical procedure, including just key portions of the medical procedure. For example, the condensed version may include an excerpt such as video 11000A extending from time *t₂* until time *t₃.* Additionally, or alternatively, the condensed version may include an excerpt such as video 11000B extending from time *t₄* until time *t₅.* The condensed version may further include an excerpt such as video 11000C extending from time *t₆* until time *t₇.* In some examples, the condensed version may include one continuous segment such as video 11000A. In some examples, videos 11000A, 11000B, and 11000C (collectively, "videos 11000") may be added together in any combination by processing circuitry 9004 to include any portion of the duration of the medical procedure less than the total duration of the medical procedure. In some examples, as illustrated, videos 11000 may individually include any portion of the medical procedure less than the entire length of the medical procedure.

Furthermore, in addition or in the alternative, the condensed version of the imaging data may include one or more images 11002A, 11002B (collectively, "images 11002"). In some examples, 11002A may correspond to a "before" picture and 11002B may correspond to an "after" picture. In this way, a clinician may be enabled to curate a shortened video and/or before vs. after still images along with key case notes, and share the curated portions of the medical procedure elegantly and efficiently with the second clinician (e.g., a referring interventional cardiologist), someone who may be too busy to consult during an entire medical procedure.

In some examples, processing circuitry 9004 may be configured to receive user input to begin video 11000A at time *t₂*. excerpt of received imaging data from the cardiac catheterization imaging data. In some examples, processing circuitry 9004 is configured to receive user input to end video 11000A at time *t₃.* In some examples, processing circuitry 9004may be configured to receive an audio command to receive user input to begin and/or end the video excerpt.

In some examples, processing circuitry 9004 may be configured to receive user input to begin the video excerpt, and further configured to output for display via display device 9010 a user interface to present an option to a clinician to begin the video excerpt of the received imaging data. In some examples, processing circuitry 9004 may be configured to execute a computer vision model 2024 (FIG. 2), alone or in combination with machine learning model 2022 (FIG. 2), to recognize a patient condition (e.g., a lesion) or an intraprocedural event (e.g., a critical timeframe such as crossing a lesion with a medical instrument). In some examples, processing circuitry 9004 may be configured to store the condensed version in memory 9002. In some examples, processing circuitry 9004 may be configured to upload the condensed version of the received imaging data to server 9060.

In some examples, processing circuitry may be configured to receive from first computing device 9050 associated with the first clinician that is performing a medical procedure, a representation of user input to request a consult from a second clinician that is located remotely from the first clinician. Processing circuitry 9004 may establish, responsive to receiving the representation of user input to request the consult, a communication session between first computing device 9050 associated with the first clinician and second computing device 9058 associated with the second clinician. Processing circuitry may stream, via the communication session, the condensed version of the imaging data from imager 9040.

In some examples, as described above with respect to the FIG. 9 example in which the medical procedure is streamed continuously in real time, processing circuitry 9004 may be configured to determine a permission state of the second clinician, and redact, prior to streaming and responsive to determining that the permission state of the second clinician is below a threshold permission state, personal health information from the condensed version of data captured by imager 9040.

The condensed version of imaging data may function as a "highlight reel" of the medical procedure that can be quickly shared and viewed by a consulting physician. Accordingly, video 11000A may include a video representation of first particular events, such as the crossing of a first lesion, and processing circuitry 9004 may be configured generate, based on the imaging data from imager 9040, a second condensed version of the imaging data as video 11000B, which may include images corresponding to second particular events of the cardiac catheterization medical procedure, such as the crossing of a second lesion. Processing circuitry 9004 may be configured to receive case notes associated with the medical procedure and may be configured to associate the received case notes with the condensed version of the imaging data.

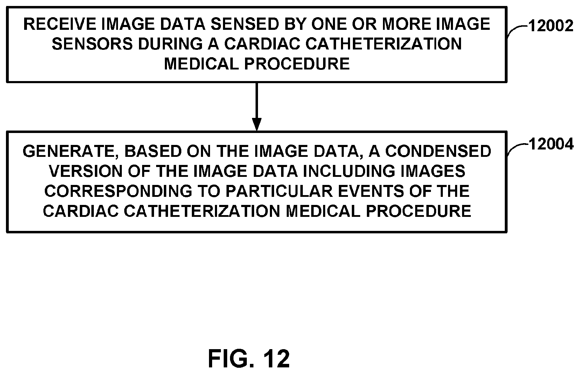

FIG. 12 is a flow diagram illustrating example techniques for generating a condensed version of imaging data sensed by one or more image sensors according to one or more aspects of the present disclosure. While described herein with respect to system 9000 of FIG. 9 and the time diagram of FIG. 11, the techniques of FIG. 12 may be implemented other systems such as system 1000 (FIG. 1), system 6000 (FIG. 6) or any combination of devices of system 9000 capable of performing such techniques.

Processing circuitry 9004 may receive imaging data sensed by imager 9040 during a medical procedure (12002). Processing circuitry 9004 may generate, based on the sensed imaging data, a condensed version of the imaging data from imager 9040, the condensed version of the imaging data including images corresponding to particular events of the cardiac catheterization medical procedure (12004). In some examples, processing circuitry 9004 may generate the condensed version of the imaging data by generating a video excerpt 11000 (FIG. 11). In some examples, processing circuitry 9004 may generate the video excerpt by receiving user input to begin and/or end video excerpt 11000 of received imaging data from the cardiac catheterization imaging data. In some examples, processing circuitry 9004 may receive an audio command from a clinician to begin and/or end video excerpt 11000.

The clinician may be presented an option to begin the condensed version or an excerpt of the condensed version through a user interface at display device 9010, output for display by processing circuitry 9004. To output for display the user interface including the option to begin the condensed version or video excerpt 11000A of the condensed version, processing circuitry 9004 may execute computer vision model 2024 (FIG.2) and/or machine learning model 2022 (FIG. 2) to recognize a patient condition or an intraprocedural event in received imaging data. Accordingly, these advanced features of system 9000 may, in some examples, assist a clinician in recognizing a situation in which a consult from a second clinician may be valuable. Responsive to a representation of user input to request a consult from a second clinician that is located remotely from the first clinician, processing circuitry 9004 may establish a communication session between a computing device associated with the first clinician and a computing device associated with the second clinician and stream the condensed version of the imaging data. Prior to establishing the communication session, processing circuitry 9004 may determine that the second clinician does not have the requisite authority to view the patient personal health information, and may redact PHI from videos 11000 and images 11002 which make up the condensed version of the imaging data.

Processing circuitry 9004 may capture key events (e.g., videos 11000A, 11000B, 11000C) of the medical procedure proceeding along time arrow A of Fig. 11. In some examples, each video 11000A of videos 11000 may represent a different particular event, and processing circuitry may capture a different set of particular events in video 11000B at a different time. Processing circuitry 9004 may upload, label, and or store videos 11000A, 11000B, and/or 11000C together or separately in memory 9002 or server 9060. In some examples, processing circuitry 9004 may associate case notes associated with the medical procedure performed, and further associate the received case notes with the condensed version of the imaging data.

FIG. 13A and 13B are example conceptual screenshots illustrating an example representation of imaging data 13700A, 13700B at a user interface associated with second computing device 9058 (FIG. 9), which is associated with a second, remote clinician. FIG. 13A illustrates an example screenshot including identifying elements 13702, 13704. Identifying elements 13704 include personal health information related to the patient undergoing the medical procedure, which may only be shared with clinicians and other employees meeting a threshold permission state. A clinician may need to be, for example, an employee or affiliate of the hospital where the medical procedure is taking place to gain the threshold permission state, meaning that system 9000 may not be used with certain clinicians acting as the second clinician who is located remotely because the imaging data to be shared via the communication session may be gathered with patient personal health information or information that the first clinician does not wish to share overlayed or otherwise attached to the imaging data. Further, the de-identified version of the imaging data may be shared to other interested parties as a teaching tool or reputation tool, for example, on a social network.

FIG. 13B illustrates the example screenshot of FIG. 13A including the representation of imaging data 13700B with identifying elements 13702, 13704 redacted by redaction boxes 13706. In this way, processing circuitry 9004 (FIG. 9) may be configured to receive imaging data from imager 9040 (FIG. 9), which may include one or more identifying elements (13702, 13704) and generate a de-identified version (FIG. 13B) of the imaging data which does not include identifying elements 13702, 13704. Processing circuitry 9004 (FIG. 9) configured to not include identifying elements 13702, 13704 may allow the imaging data from imager 90004 to be safely shared to interested parties.

Although identified elements 13702, 13704 are described with respect to FIG. 13B as blocked out by redaction boxes 13706, processing circuitry 9004 (FIG. 9) may be configured to not include identifying elements 13702, 13704 in the de-identified version in other ways. Processing circuitry 9004 (FIG. 9) may be configured to redact, remove, obfuscate, or render illegible identifying elements 13704 which include PHI to generate the de-identified version of the imaging data. For example, since imager 9040 (FIG. 9) may apply a text overlay containing identifying elements to sensed imaging data, processing circuitry 9004 (FIG. 9) may be configured to scan imaging data from imager 9040 (FIG.9) for a text overlay, identify a text overlay, and redact, remove, obfuscate, or render illegible the text overlay.

In some examples, processing circuitry 9004 (FIG. 9) may be configured to upload the de-identified version (FIG. 13B) of the imaging data to server 9060. In some examples, processing circuitry 9004 (FIG. 9) may be configured to post the de-identified version (FIG. 13B) of the imaging data on a social network, such as a physician-only social network.

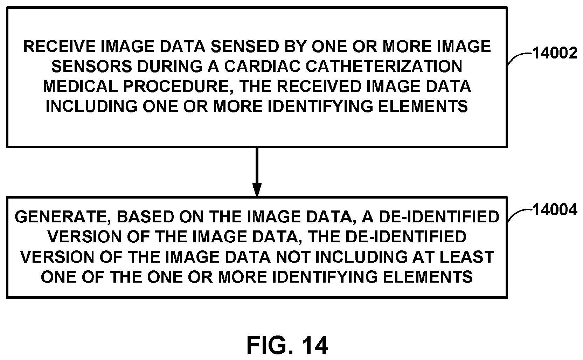

FIG. 14 is a flow diagram illustrating example techniques for generating a de-identified version of imaging data sensed by one or more image sensors during a medical procedure according to one or more aspects of the present disclosure. While described herein with respect to system 9000 of FIG. 9 and versions of the imaging data of FIGS. 13A and 13B, the techniques of FIG. 14 may be implemented other systems such as system 1000 (FIG. 1), system 6000 (FIG. 6) or any combination of devices of system 9000 capable of performing such techniques.

Processing circuitry 9004 may receive imaging data from imager 9040 during a medical procedure, the received imaging data including one or more identifying elements 13702, 13704 (14002). Processing circuitry 9004 may generate, based on the imaging data from imager 9040, a de-identified version (FIG. 13B) of the imaging data, the de-identified version of the imaging data not including identifying elements 13702, 13704. Identifying elements 13704 may include personal health information, and processing circuitry 9004 may generate the de-identified version (FIG. 13B) by redacting, removing, obfuscating, or rendering illegible the personal health information to generate the de-identified version (FIG. 13B) of the imaging data. In some examples, processing circuitry 9004 may scan imaging data from the one or more image sensors for a text overlay, identifying a text overlay; and redact remove, obfuscate, or otherwise render the text overlay illegible.

In some examples, processing circuitry 9004 may upload the de-identified version (FIG. 13B) of the imaging data to a server. In some examples, processing circuitry 9004 may post the de-identified version of the imaging data on a social network such as a physician-only social network. In some examples, the imaging data from imager 9040 may include video data, fluoroscopy data, or both.

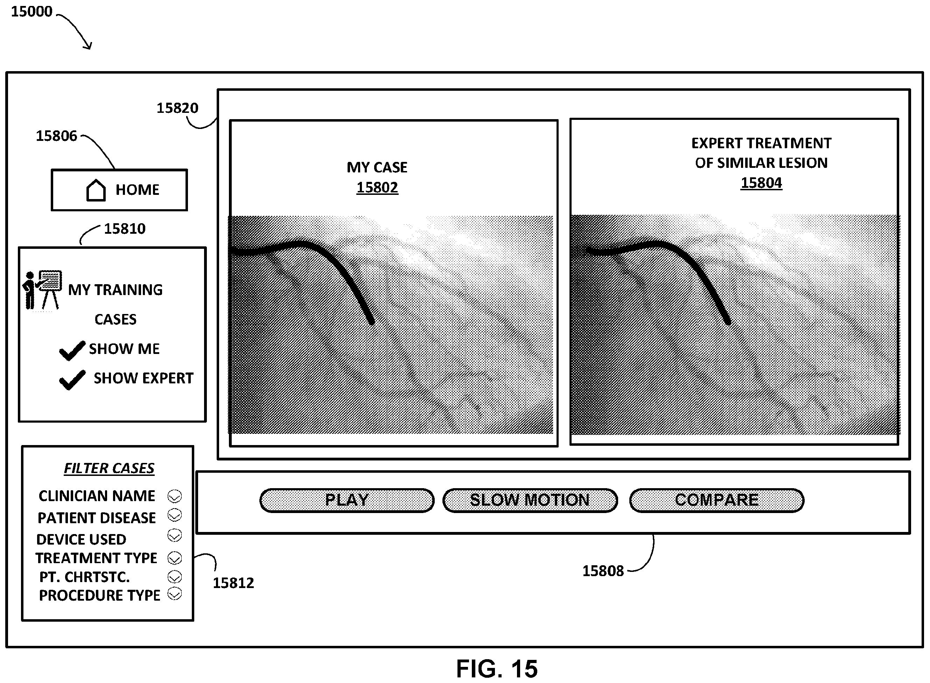

FIG. 15 is a screenshot illustrating an example user interface 15000 for using an example medical system of the present disclosure for educational purposes, according to one or more aspects of the present disclosure. User interface 15000 may be a user interface present at a display of more or more devices of system 9000 of FIG. 9. Processing circuitry 9004 (FIG. 9) may be configured to output user interface 15000 to any suitable display, such as display device 9010 (FIG. 9), a display associated with first computing device 9050 (FIG. 9), a display associated with second computing device 9058, or another display device.

Processing circuitry 9004 (FIG. 9) may be configured to output for display at user interface 15000 a home button 15806, viewing window 15820, training menu 15810, filtration menu 15812, and operations menu 15808.

Viewing window 15820 may display a representation of imaging data from imager 9040 (FIG. 9) or another imager captured during a first medical procedure 15802, illustrated as "MY CASE" in FIG. 15. In some examples, processing circuitry 9004 (FIG. 9) may be configured to automatically display a most recently completed medical procedure stored in memory 9002 (FIG. 9) as the first medical procedure 15802. In some examples, processing circuitry 9004 (FIG. 9) may be configured to receive user input to find and select first medical procedure stored within memory 9002 (FIG. 9) to output for display as the first medical procedure. Processing circuitry 9004 (FIG. 9) may be configured to output for display a plurality of dropdown menus in filtration menu 15812 to help a clinician find and select a first medical procedure. Additionally, or alternatively, processing circuitry 9004 (FIG. 9) output for display a search bar, and include search functionality that a clinician may use to identify and select first medical procedure 15802 from within memory 9002 (FIG. 9).

Viewing window 15820 may also display a representation of imaging data from a second medical procedure 15804, illustrated as "EXPERT TREATMENT OF SIMILAR LESION." The representation of imaging data from the second medical procedure 15804 may be stored in memory 9002 (FIG. 9). Processing circuitry 9004 (FIG. 9) may be configured to execute computer vision model 2024 (FIG. 2) and/or machine learning model 2024 (FIG. 2) to identify and/or select second medical procedure 15804 from a plurality of medical procedures to output for display with first medical procedure 15802. Processing circuitry 9004 (FIG. 9) configured to display first medical procedure 15802 and second medical procedure 15804 may be an advantageous learning tool for a clinician to compare lesions, techniques, treatment strategies, and the like between first medical procedure 15802 and second medical procedure 15804. In some examples, a clinician may select a medical procedure that they have recently performed as first medical procedure 15802, and compare their strategy to another clinician's strategy in a similar case, where the identification of the similar case is enabled by computer vision model 2024 and/or machine learning model 2022.

As describe above, processing circuitry 9004 (FIG. 9) may execute computer vision model 2022 using propensity matching to identify and select a medical procedure for display as second medical procedure 15804. In some examples, propensity matching may include using processing circuitry 9004 (FIG. 9) to analyze imaging data sensed by imager 9040 (FIG. 9) to determine a type of lesion, a sub-type of lesion, or otherwise classify the lesion (e.g., provide a score or other identifier), or the like, based on the determined characteristics of the lesion. Characteristics of the lesion may include, for example, the lesion type (e.g., bifurcation lesion), lesion diameter, the degree of stenosis, the degree of calcification, vessel take-off angles, etc. The classification of the lesion may be such that lesions having a large degree of similarity are classified the same or close to each other. Processing circuitry may be configured to display as second medical procedure 15804 a medical procedure that includes a lesion within the same classification or a similar classification as a lesion present within first medical procedure 15802.

In some examples, processing circuitry 9004 (FIG. 9) may be configured to use computer vision model 2024 (FIG. 2) and/or machine learning model 2022 (FIG. 2) to perform propensity matching by comparing imaging data from the first medical procedure 15802 to a plurality of the plurality of medical procedures stored in memory 9002 (FIG. 9), and select the most similar medical procedure from the plurality of medical procedures stored in memory 9002 (FIG. 9) to output for display with first medical procedure 15802 based on the similarity of one or more patient conditions of medical procedure 15802 to the second medical procedure. In some examples, first medical procedure 15802 may be compared to every medical procedure of the plurality of medical procedures stored in memory 9002 (FIG. 9), and the most similar medical procedure in one or more ways may be selected to output for display as second medical procedure 15804. Alternatively, a threshold level of similarity may be set (e.g., a patient condition including a legion of the same classification) and processing circuitry 9004 (FIG. 9) may output any one of the medical procedures meeting the threshold to output for display as second medical procedure 15804. In some examples, processing circuitry 9004 may be configured to compare medical procedures stored within memory 9002 that include lesions within the vasculature of a patient, and may compare lesions based upon lesion characteristics including at least one of a lesion length, shape, geometry, location, degree, vessel take-off angle, or percentage calcification.

Processing circuitry 9004 (FIG. 9) may also output for display at user interface 1600 filtration menu 15812, which may enable a clinician to filter medical procedures stored in memory 9002 (FIG. 9) in one or more ways. Filters may be applied to narrow the candidate medical procedures for one or both of first medical procedure 15802 and/or second medical procedure 15804. Filtration menu 15812, as illustrated, may present a clinician an option to filter by one or more of a patient characteristic, a patient condition, a medical tool or equipment used during the cardiac catheterization medical procedure, an operating clinician, a treatment or class of treatments used. Additionally or alternatively, filtration menu 15812 may present a clinician an option to filter by a hospital type, a sequence of tools or treatments used, or a length of procedure. In some examples, a patient characteristic may include a patient age, height, weight, sex, disease, or diagnosis. In some examples, medical device, tool, or equipment used may include a catheter tip size or geometry.

In some examples, the imaging data forming a representation of first medical procedure 15802 may be video data. In some examples the imaging data forming a representation of second medical procedure 15804 may be video data. Although illustrated in FIG. 15 as adjacent to one another, in some examples, processing circuitry 9004 (FIG. 9) may be configured to cause a display to overlay the video data from second medical procedure 15804 over video data first medical procedure 15802, or vice versa, such that the videos are on top of one another within viewing window 15820. Viewing the medical procedures in this way may be advantageous to a clinician to more closely analyze the position of a lesion, the speed of an instrument through the vasculature, or other parameters, or the like. Although described herein as two different medical procedures for ease of description, in some examples processing circuitry 9004 (FIG. 9) may be configured to output more than two medical procedures for display within viewing window 15820, such as three medical procedures, or four medical procedures, or more.

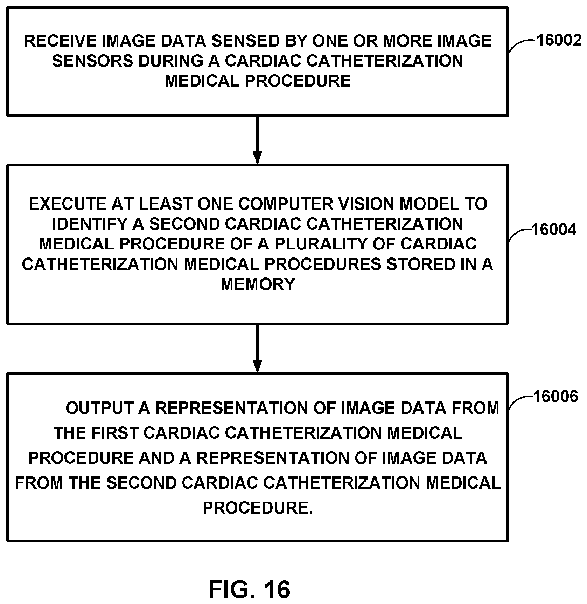

FIG. 16 is a flow diagram illustrating example techniques for using an example medical system of the present disclosure for educational purposes. While described herein with respect to system 9000 of FIG. 9 and user interface 15000 of FIG. 15, the techniques of FIG. 16 may be implemented other systems such as system 1000 (FIG. 1), system 6000 (FIG. 6) or any combination of devices of system 9000 capable of performing such techniques.

Processing circuitry 9004 (FIG. 9) may receive imaging data from imager 9040 (FIG. 9) during a medical procedure 15802 (FIG. 15) (16002). Processing circuitry 9004 (FIG. 9) may execute a computer vision model 2024 (FIG. 2) and/or machine learning model 2022 (FIG. 2) identify and/or select second medical procedure 15804 (FIG. 15) of a plurality of medical procedures stored in memory 9002 (FIG. 9) (16004). Processing circuitry 9004 (FIG. 9) may output, for display at display device 9010 (FIG. 9), a representation of imaging data from first medical procedure 15802 (FIG. 15) and a representation of imaging data from second medical procedure 15804 (FIG. 15) (16006).

In some examples, second medical procedure 15804 (FIG. 15) includes a similar patient condition as first medical procedure 15802 (FIG. 15). In some examples, processing circuitry 9004 (FIG. 9) may execute computer vision model 2024 (FIG. 2) using propensity matching to find second medical procedure 15804 (FIG. 15) in memory 9002 (FIG. 9).

In some examples, processing circuitry 9004 (FIG. 9) may use propensity matching by comparing imaging data from first medical procedure 15802 (FIG. 15) to a plurality of the plurality of medical procedures stored in memory 9002 (FIG. 9), and selecting second medical procedure 15804 (FIG. 15) from the plurality of medical procedures stored in memory 9002 (FIG. 9) to output for display with first medical procedure 15802 (FIG. 15) based on the similarity of one or more characteristics of the first medical procedure 15802 (FIG. 15) to second medical procedure15804 (FIG. 15).

In some examples, the patient condition may be a lesion, and processing circuitry 9004 (FIG. 9) may compare one or more lesion characteristics from first medical procedure 15802 (FIG. 15) to a plurality of medical procedures stored in memory 9002 (FIG. 9), and identify and select second medical procedure 15804 (FIG. 15) from the plurality of cardiac catheterization medical procedures stored in memory 9002 (FIG. 9) by finding the most similar lesion based on the one or more lesion characteristics from the plurality of medical procedures stored in memory 9002 (FIG. 9). In some examples, wherein the one or more lesion characteristics include at least one of a lesion length, shape, geometry, location, degree, vessel take-off angle, and/or degree of calcification.

In some examples, processing circuitry 9004 (FIG. 9) may cause user interface 15000 (FIG. 15) to present an option to a clinician to filter the plurality of medical procedures stored in memory 9002 (FIG. 9) in one or more ways. In some examples, processing circuitry 9004 (FIG. 9) may cause user interface 15000 (FIG. 15) to present the clinician an option to filter by one or more of a patient characteristic, a patient condition, a medical tool or equipment used during the cardiac catheterization medical procedure, an operating clinician, a treatment or class of treatments used, a hospital type, a sequence of tools or treatments used, and/or a length of procedure. In some examples wherein the patient characteristic includes at least one of a patient age, height, weight, sex, disease, or diagnosis. In some examples wherein the medical tool or equipment used includes one or more of a catheter tip size or geometry.

In some examples, processing circuitry 9004 (FIG. 9) may cause user interface 15000 (FIG. 15) to display first medical procedure 15802 (FIG. 15) and second medical procedure 15804 (FIG. 15) within viewing window 15820 (FIG. 15). In some examples, processing circuitry 9004 (FIG. 9) may cause user interface 15000 (FIG. 15) to overlay video data from second medical procedure 15804 (FIG. 15) over video data from first medical procedure 15802 (FIG. 15) in viewing window 15820 (FIG. 15). Alternatively, in some examples, video from the two medical procedures may be displayed adjacent to each other within viewing window 15820 (FIG. 15).

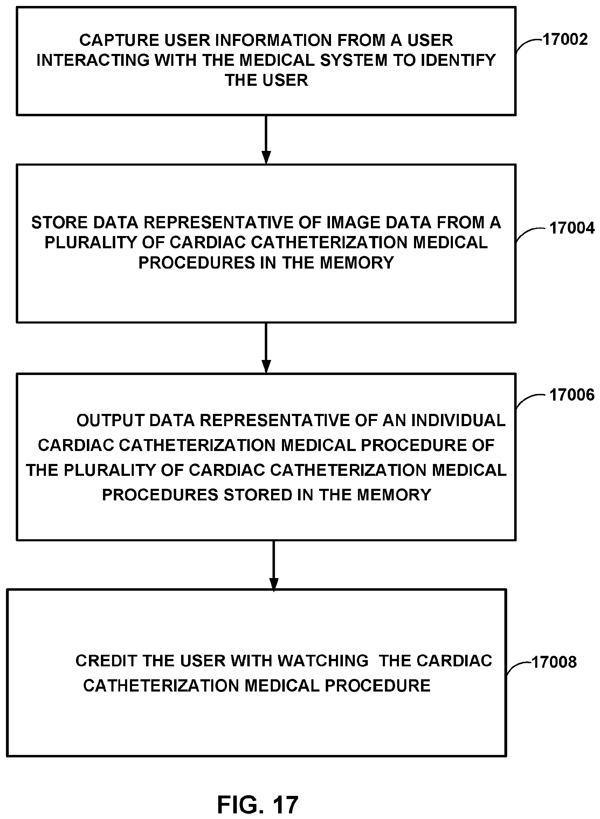

FIG. 17 is a flow diagram illustrating example techniques for using an example medical system of the present disclosure for educational purposes, according to one or more aspects of the present disclosure. While described herein with respect to system 9000 of FIG. 9 and user interface 15000 of FIG. 15, the techniques of FIG. 16 may be implemented other systems such as system 1000 (FIG. 1), system 6000 (FIG. 6) or any combination of devices of system 9000 capable of performing such techniques.

Processing circuitry 9004 (FIG. 9) may capture user information from a user interacting with the medical system to identify the user (17002). In some examples, processing circuitry 9004 (FIG. 9) may output for display a user log-in page to capture user information from a user interacting with the medical system to identify the user.

Processing circuitry 9004 may store data representative of imaging data from a plurality of medical procedures in memory 9002 (FIG. 9) (17004). Processing circuitry 9004 (FIG. 9) may output data representative of an individual cardiac catheterization medical procedure of the plurality of cardiac catheterization medical procedures stored in memory 9002 (FIG. 9) (17006). Subsequent to outputting for display the data representative of the individual medical procedure, credit the user with watching the individual cardiac catheterization medical procedure (17008).

In some examples, crediting the user with watching the excerpt of the medical procedure may include awarding a user at least a portion of a continuing medical education (CME) credit. In some examples, processing circuitry 9004 (FIG. 9) may integrate with a credentialling body, and as part of awarding a user at least a portion of a CME credit, processing circuitry 9004 (FIG. 9) may report a name of the user to the credentialling body. In some examples, processing circuitry 9004 (FIG. 9) may cause user interface 15000 (FIG. 15) associated with display device 9010 (FIG. 9), an option to a user to filter the plurality of medical procedures in one or more ways. In some examples, processing circuitry 9004 (FIG. 9) may cause user interface 15000 (FIG. 15) to present a plurality of drop-down menus for display as filtration menu 15812 (FIG. 15).

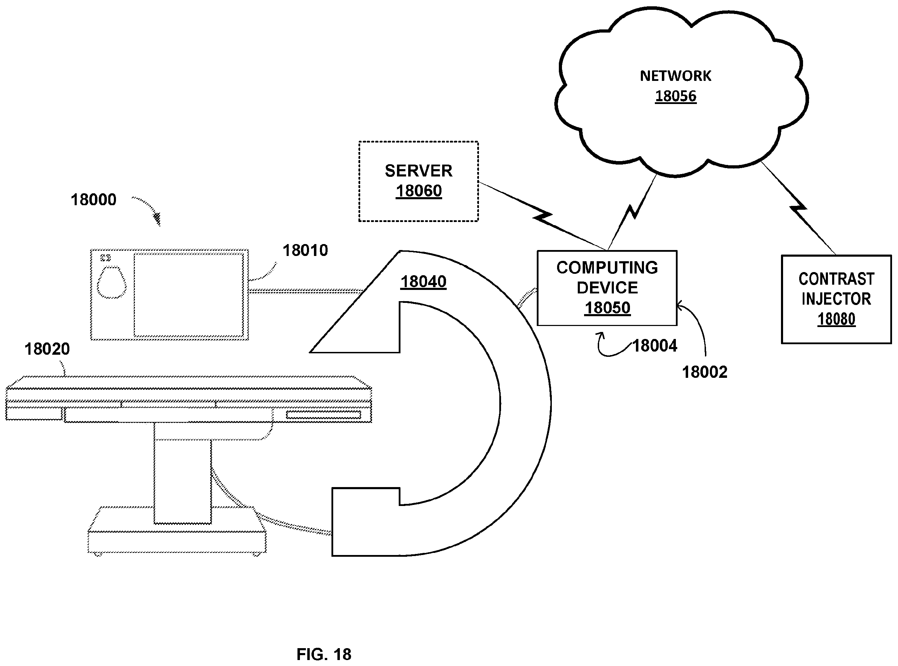

FIG. 18 is a schematic perspective view of example medical system 18000. Medical system 18000 may be an example of medical system 1000 of FIG.1 and/or medical system 9000 of FIG. 6. Medical system 18000 of FIG. 18 is similar to medical system 9000 of FIG. 9, differing as described below, where similar reference numbers indicate similar elements. Medical system 18000 may perform various contrast management functions to support a Cath Lab procedure.

System 18000 includes a display device 18010, a table 18020, an imager 18040, a computing device 18050, and a server 18060. System 18000 may be an example of a system for use in a Cath lab. In some examples, system 18000 may include other devices, such as additional devices depicted in FIG. 1, which are not shown in FIG. 18 for simplicity purposes. System 18000 may be used during a Cath Lab procedure session to diagnose and/or intervene in cardiovascular issues of a patient.

Computing device 18050 may be associated with a clinician, who may be located in the Cath Lab during the medical procedure. Computing device 18050 may be an example of computing device 6050 or 6052 (FIG. 6) and may include, for example, an off-the-shelf device, such as a laptop computer, desktop computer, tablet computer, smart phone, or other similar device. Computing device 18050 includes memory 18002 and processing circuitry 18004.

While processing circuitry 18004 appears in computing device 18050 in FIG. 18, in some examples, features attributed to processing circuitry 18004 may be performed by processing circuitry of any of computing device 18050, imager 18040, server 18060, other elements of system 18000, or combinations thereof. In some examples, one or more processors associated with processing circuitry 18004 in computing device 18050 may be distributed and shared across any combination of computing device 18050, imager 18040, server 18060, network 18056, and display device 18010. Additionally, in some examples, processing operations or other operations performed by processing circuitry 18004 may be performed by one or more processors residing remotely, such as one or more cloud servers or processors, each of which may be considered a part of computing device 18050.

System 18000 includes network 18056, which is a suitable network such as a local area network (LAN) that includes a wired network or a wireless network, a wide area network (WAN), a wireless mobile network, a Bluetooth network, or the internet. In some examples, network 18056 may be a secure network, such as a hospital network, which may limit access by users.

As discussed above, imager 18040 may be an angiography imager or other imaging device, and may be used to image the patient's body during the procedure to visualize characteristics and locations of lesions inside the patient's body. Imager 9040 may be any type of imaging device, such as a CT device, an MRI device, a fluoroscopic device, a PET device, an ultrasound device, or the like.

During a Cath Lab procedure, contrast may be injected into a patient's vasculature. The contrast may enhance the appearance of blood and/or other components in imaging data captured by imager 18040. In some examples, the contrast may be manually injected into the vasculature by a clinician (e.g., cardiologist, nurse, or other) via a syringe. In other examples, system 18000 may include a contrast injector (e.g., a power injector) that may automatically inject/dispense the contrast.

In general, the amount of contrast used in a Cath Lab procedure is a tradeoff between using enough contrast to make the resulting images useful, and not using too much contrast so as to cause undesirable side effects. For instance, the use of too much contrast may result in a condition known as contrast induced nephropathy (CIN).

The clinician performing the Cath Lab procedure may have a general target for how much contrast they plan to use for the procedure. However, tracking actual versus planned contrast usage may undesirably impact the clinician's workload during the procedure.

In accordance with one or more aspects of this disclosure, system 18000 may provide automatic tracking of contrast usage. For instance, computing device 18050 may determine a cumulative amount of contrast used during a cardiac catheterization lab (Cath Lab) procedure, and cause display device 18010 to display, during the Cath Lab procedure, a graphical representation of the cumulative amount of contrast used. As such, system 18000 may enable the clinician to quickly determine how much contrast has been used (e.g., at a glance).

The graphical representation displayed at display device 18010 may be in any suitable form. Examples include, but are not limited to, graphs (e.g., bar graphs, pie charts, line graphs, etc.), textual representations (e.g., numbers on the display), or any other representation. As one example, the graphical representation may include a graph that has a plot of an amount of contrast used over time. As another example, the textual representation may include a percentage of contrast used relative to an expected or maximum amount of contrast.

In some examples, computing device 18050 may provide contextual data to assist the clinician in better understanding the amount of contrast that has been used. For instance, computing device 18050 may include, in the graphical representation, an amount of contrast expected to have been used by a current point in the Cath Lab procedure. This may further assist the clinician in determining whether contrast administration should be slowed down (e.g., where the actual amount used is greater than the expected amount), or whether additional contrast buffer is available (e.g., where the actual amount used is less than the expected amount).

Computing device 18050 may output the comparisons between expected and actual contrast amounts at various temporal scopes. As one example, computing device 18050 may output a whole procedure comparison of contrast usage. For instance, computing device 18050 may cause display device 18010 to display a graphical representation of a comparison between a cumulative amount of contrast used so far during the cardiac catheterization lab procedure and a total amount of contrast expected/predicted to use during the entire procedure. As another example, computing device 18050 may output a step-wise comparison of contrast usage. For instance, where performance of the Cath Lab procedure includes performance of a plurality of steps (e.g., navigating devices to lesions, utilizing devices to intervene in lesions, measuring post-intervention blood flow, etc.), computing device 18050 may determine separate target contrast dosages for different steps and display graphical representations of comparisons between target contrast dosages for steps and corresponding amounts of contrast used during performance of the steps.

In general, the cadence of contrast usage may not be linearly distributed throughout the Cath Lab procedure. Performance of certain steps may utilize more contrast than other steps. As such, a linear representation of target contrast usage relative to steps (e.g., that 37.5% of the total contrast is expected by used by the end of step 3 of 8) may not be useful to the clinician. In accordance with one or more aspects of this disclosure, the target contrast dosages for the steps may be different. For instance, a target contrast dosage for a navigation step may be lower than a target contrast dosage for a measurement step. In this way, system 18000 may provide higher quality contrast usage guidance to the clinician.

There may be a total planned contrast dosage at the start of the Cath Lab procedure (e.g., a sum of the target contrast dosages for all of the steps). However, in some cases, events may occur that result in the clinician wanting to administer more contrast than the total planned contrast dosage. As discussed above, CIN may result from too much contrast being administered to a patient. However, different patients may be able to tolerate different amounts of contrast without experiencing CIN. As such, it may be desirable for the clinician to be able to determine how much contrast can still be administered to the patient without causing CIN.

In accordance with one or more aspects of this disclosure, computing device 18050 may obtain a maximum contrast dosage for the Cath Lab procedure and output a comparison between the cumulative amount of contrast used and the maximum contrast dosage. For instance, computing device 18050 may cause display device 18010 to display a graphical representation of a comparison between the cumulative amount of contrast used and the maximum contrast dosage (e.g., 80 cubic-centimeters (cc) used of 120 cc maximum dosage). In this way, computing device 18050 may enable the clinician to quickly determine how much additional contrast may be used without causing CIN.

As discussed above, computing device 18050 may determine amounts of contrast used. In general, computing device 18050 may determine the amounts of contrast used via any suitable input source. As one example, computing device 18050 may receive, via a user interface, a manual entry (e.g., by the clinician or another person present, such as nurse) of contrast usage. For instance, the manual entry may indicate how many syringes of a certain capacity (e.g., 20cc) have been used. As another example, computing device 18050 may receive, via one or more sensors (e.g., a flow meter), data that represents contrast usage. For instance, computing device 18050 may receive the data from sensors integrated in contrast injector 18080, or sensors in-line between contrast injector 18080 and the patient. In some examples, the connection between the sensors and computing device 18050 may be wireless (e.g., BLUETOOTH, Wi-FI, etc.). The data received may indicate a contrast flow rate (e.g., cc/min) or may indicate the cumulative amount of contrast used (e.g., cc). Where the data indicates the contrast flow rate, computing device 18050 may determine, based on the contrast flow rate (and historical flowrate data for the procedure), the cumulative amount of contrast used (e.g., integrate the flow rate data).

As discussed above, computing device 18050 may utilize expected/predicted amounts of contrast (e.g., when generating graphical representations of actual contrast usage compared to expected contrast usage). In some examples, the expected amounts may be generic non-patient specific amounts (e.g., standards amounts for navigation steps, measurement steps, etc.). In other examples, the expected amounts may be patient-specific. For instance, computing device 18050 may determine a patient-specific predicted amount of contrast based on attributes of a current patient. In some examples, the attributes may include imaging data of the current patient captured prior to the cardiac catheterization lab procedure (e.g., diagnostic angiogram imaging data). The computing device 18050 determines, based on the imaging data, a classification of the current patient (e.g., using a machine learning model, such as described herein). Computing device 18050 then determines, based on amount of contrast used for other patients having the classification, the predicted amount of contrast for the current patient. As such, computing device 18050 may predict the amount of contrast for the current patient based on amounts of contrast used in similar cases.

In some examples, computing device 18050 may perform clinician agnostic contrast prediction. For instance, computing device 18050 may predict the amount of contrast for the current patient regardless of attributes of the clinician that is to perform the procedure. In other examples, computing device 18050 may perform clinician specific contrast prediction. For instance, computing device 18050 may predict the amount of contrast for the current patient based on attributes of the clinician that is to perform the procedure.

In some examples, computing device 18050 may generate the clinician specific contrast amount by adjusting the clinician agnostic amount. As one example, computing device 18050 may determine that the clinician that is to perform the procedure uses X% less than the clinician agnostic predicted amount. In such examples, computing device 18050 may reduce the clinician agnostic predicted amount by X% to generate the clinician specific predicted amount. Similarly, computing device 18050 may increase the clinician agnostic predicted amount if the clinician typically uses more than the clinician agnostic predicted amount.

In some examples, computing device 18050 may generate the clinician specific contrast amount by predicting based on similar cases performed by the specific clinician. For instance, computing device 18050 may determine the predicted amount based on amounts of contrast actually used by the specific clinician in similar cases.

As noted above, computing device 18050 may obtain a maximum contrast dosage for the current patient and may predict expected contrast usage for the current patient. In some examples, computing device 18050 may output a warning or other indication to the clinician (e.g., during a planning phase) if the predicted contrast usage for the current patient is greater than the maximum contrast dosage for the current patient. As such, computing device 18050 may provide the clinician with advance warning such that the clinician may modify their plan in advance of actually starting the procedure, which may avoid undesirable situations.

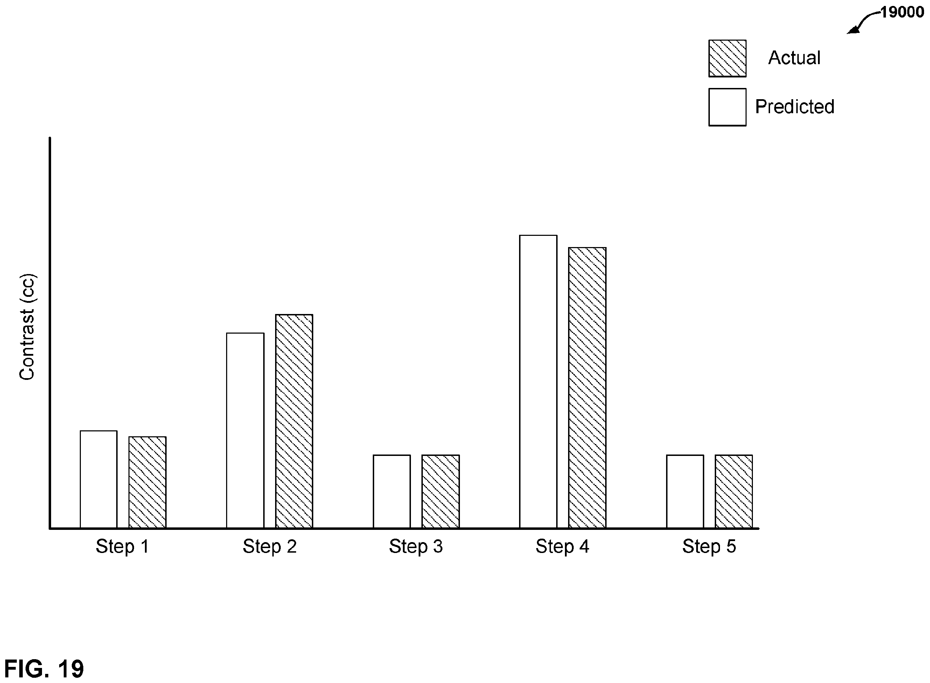

FIG. 19 is a conceptual diagram illustrating an example graphical user interface (GUI) that includes contrast usage data, in accordance with one or more aspects of this disclosure. GUI 19000 of FIG. 19 may be displayed at a display device, such as display device 18010 of FIG. 18. GUI 19000 is only one example of a GUI that includes contrast usage data, and other arrangements are contemplated.

As shown in FIG. 19, GUI 19000 includes contrast usage data for five steps of a Cath Lab procedure. A computing device, such as computing device 18050, may update GUI 19000 as the Cath Lab procedure progresses. For instance, GUI 19000 may initially include just the predicted bar for Step 1. Then, as contrast is administered or at the end of performance of Step 1, GUI 19000 may update to include the actual bar. As can be seen in the example of FIG. 19, the amount of contrast actually used in Step 1 is slightly less than the predicted amount. By contrast, the amount of contrast actually used in Step 2 is slightly more than the predicted amount. While illustrated as including five steps, GUI 19000 may include contrast information for more or fewer steps.

In some examples, GUI 19000 may omit contrast usage information for some steps. For instance, GUI 19000 may include contrast usage information for a current step, but may omit or summarize contrast usage information for previous steps. As one specific example, while Step 3 is being performed, GUI 19000 may include elements that sum the actual and predicted amounts of already completed steps (e.g., Step 1 and Step 2) and/or predicted amounts for subsequent steps (e.g., Step 4 and Step 5).

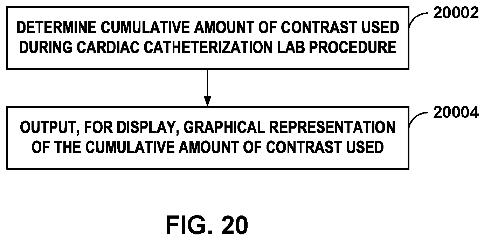

FIG. 20 is a flow diagram illustrating example techniques for providing contrast usage data, according to one or more aspects of the present disclosure. While described herein with respect to system 18000 of FIG. 18 and GUI 19000 of FIG. 19, the techniques of FIG. 20 may be implemented other systems such as system 1000 (FIG. 1), system 6000 (FIG. 6) or any combination of devices of system 18000 capable of performing such techniques.

Processing circuitry 18004 (FIG. 18) may determine a cumulative amount of contrast used during a cardiac catheterization lab (Cath Lab) procedure (20002) and output, for display, a graphical representation of the cumulative amount of contrast used

(20004). As noted above, in some examples, the graphical representation may include a comparison between the cumulative amount of contrast used and an expected amount of contrast used. This comparison may be procedure-wise, or may be step-wise (e.g., FIG. 19 illustrates such a step-wise comparison).

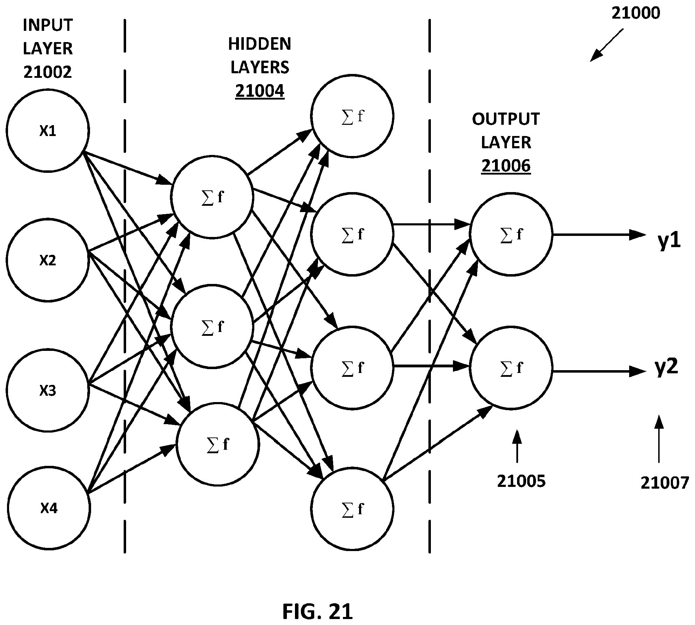

FIG. 21 is a conceptual diagram illustrating an example machine learning model according to one or more aspects of this disclosure. Machine learning model 21000 may be an example of the machine learning model(s) 7022 or any other machine learning model described herein. In some examples, machine learning model 21000 may be a part of computer vision model(s) 7024 or any other computer vision models described herein. Machine learning model 21000 may be an example of a deep learning model, or deep learning algorithm, trained to predict contrast usage. One or more of computing device 6050, computing device 7000 (or any other computing device described herein) and/or server 6060 (or any other server described herein) may train, store, and/or utilize machine learning model 21000, but other devices of system 6000 (or any other system described herein) may apply inputs to machine learning model 21000 in some examples. In some examples, other types of machine learning and deep learning models or algorithms may be utilized in other examples. For example, a convolutional neural network model of ResNet-18 may be used. Some non-limiting examples of models that may be used for transfer learning include AlexNet, VGGNet, GoogleNet, ResNet50, or DenseNet, etc. Some non-limiting examples of machine learning techniques include Support Vector Machines, K-Nearest Neighbor algorithm, and Multi-layer Perceptron.

As shown in the example of FIG. 21, machine learning model 21000 may include three types of layers. These three types of layers include input layer 21002, hidden layers 21004, and output layer 21006. Output layer 21006 comprises the output from the transfer function 21005 of output layer 21006. Input layer 21002 represents each of the input values X1 through X4 provided to machine learning model 21000. In some examples, the input values may include any of the of values input into the machine learning model, as described above. For example, the input values may include contrast usage data, procedure data, and/or other data as described above. In addition, in some examples input values of machine learning model 21000 may include additional data, such as other data that may be collected by or stored in system 6000 (or any other system described herein).

Each of the input values for each node in the input layer 21002 is provided to each node of a first layer of hidden layers 21004. In the example of FIG. 21, hidden layers 21004 include two layers, one layer having four nodes and the other layer having three nodes, but fewer or greater number of nodes may be used in other examples. Each input from input layer 21002 is multiplied by a weight and then summed at each node of hidden layers 21004. During training of machine learning model 21000, the weights for each input are adjusted to establish the relationship between predicted contrast amounts, historical contrast amounts, and actual contrast amounts. In some examples, one hidden layer may be incorporated into machine learning model 21000, or three or more hidden layers may be incorporated into machine learning model 21000, where each layer includes the same or different number of nodes.

The result of each node within hidden layers 21004 is applied to the transfer function of output layer 21006. The transfer function may be liner or non-linear, depending on the number of layers within machine learning model 21000. Example non-linear transfer functions may be a sigmoid function or a rectifier function. The output 21007 of the transfer function may be a predicted amount of contrast, and/or the like.

As shown in the example above, by applying machine learning model 21000 to input data such as imaging data 7014 and/or lesion classification(s) 7030, processing circuitry 7004 is able to determine a predicted amount of contrast. This may improve patient outcomes.

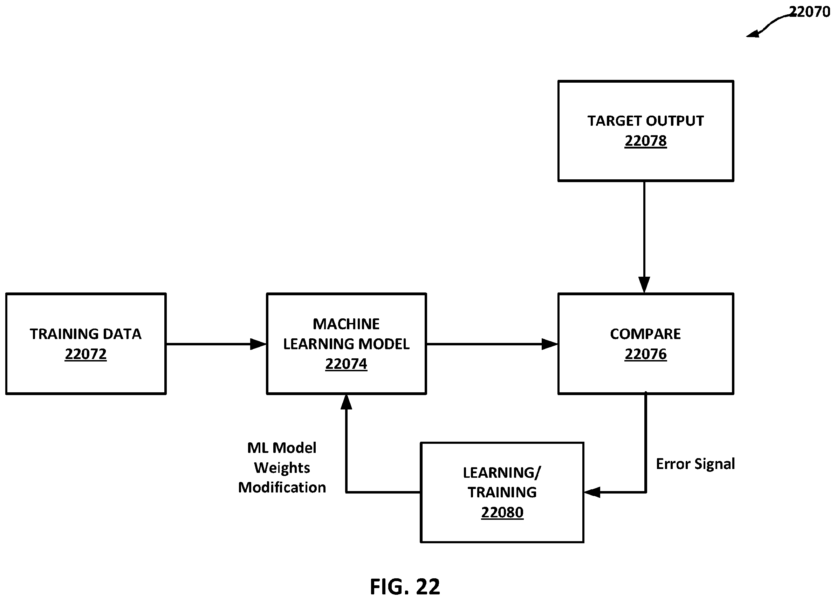

FIG. 22 is a conceptual diagram illustrating an example training process for a machine learning model according to one or more aspects of this disclosure. Process 22000 may be used to train machine learning model(s) 7022 (or any other machine learning model discussed herein) and/or computer vision model(s) 7024 (or any other computer vision model discussed herein). A machine learning model 22074 (which may be an example of machine learning model 21000, machine learning model(s) 7022 or any other machine learning model discussed herein) may be implemented using any number of models for supervised and/or reinforcement learning, such as but not limited to, an artificial neural network, a decision tree, naïve Bayes network, support vector machine, or k-nearest neighbor model, CNN, RNN, LSTM, ensemble network, to name only a few examples. In some examples, one or more of computing device 6050 (or any other computing device discussed herein) and/or server 6060 (or any other server discussed herein) initially trains machine learning model 22074 based on a corpus of training data 22072. Training data 22072 may include, for example, data collected from past medical procedures including at least one of past contrast usage data.

While training machine learning model 22074, processing circuitry 7004 (or any other processing circuitry discussed herein) may compare 22076 a prediction or classification with a target output 22078. Processing circuitry 7004 may utilize an error signal from the comparison to train (learning/training 22080) machine learning model 22074. Processing circuitry 7004 may generate machine learning model weights or other modifications which processing circuitry 7004 may use to modify machine learning model 22074. For example, processing circuitry 7004 may modify the weights of machine learning model 21000 based on the learning/training 22080. For example, one or more of computing device 6050 and/or server 6060, may, for each training instance in training data 22072, modify, based on training data 22072, the manner in which a predicted amount of contrast is determined.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The terms "controller", "processor", or "processing circuitry" may generally refer to any of the foregoing logic circuitry, alone or in combination with other logic circuitry, or any other equivalent circuitry. A control unit comprising hardware may also perform one or more of the techniques of this disclosure. Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), or electronically erasable programmable read only memory (EEPROM), or other computer readable media.

Various examples have been described.

## Claims

1. A medical system comprising:
a memory (2002); and
processing circuitry (2004) communicatively coupled to the memory (2002), the processing circuitry (2004) being configured to:
determine, at a current point of a cardiac catheterization lab procedure, a cumulative amount of contrast used during the cardiac catheterization lab procedure (5000) up to the current point;
determine a predicted amount of contrast to have been used by the current point of the cardiac catheterization lab procedure wherein, to determine the predicted amount of contrast, the processing circuitry is configured to:
determine, based on the imaging data of the current patient captured prior to the cardiac catheterization lab procedure (5000), a classification of the current patient; and
determine, based on amount of contrast used for other patients having the classification, the predicted amount of contrast for the current patient; and
output, for display (2006) and during the cardiac catheterization lab procedure (5000), a graphical representation of the cumulative amount of contrast used and the predicted amount of contrast to have been used.

2. The medical system of claim 1, wherein the cardiac catheterization lab procedure (5000) is comprised of a plurality of steps, and wherein the processing circuitry (2004) is further configured to:
determine a target contrast dosage for a first step of the plurality of steps;
determine an amount of contrast used during performance of the first step; and
output, for display (2006) and during the cardiac catheterization lab procedure (5000), a graphical representation of a comparison between the target contrast dosage for first step and the amount of contrast used during performance of the first step.

3. The medical system of claim 2, wherein the processing circuitry (2004) is further configured to:
determine a target contrast dosage for a second step of the plurality of steps, the target contrast dosage for the second step being different than the target contrast dosage for the first step;
determine an amount of contrast used during performance of the second step; and
output, for display (2006) and during the cardiac catheterization lab procedure (5000), a graphical representation of a comparison between the target contrast dosage for second step and the amount of contrast used during performance of the second step.

4. The medical system of claim 3, wherein the target contrast dosage for the second step is greater than the target contrast dosage for the first step.

5. The medical system of claim 4, wherein the first step comprises a navigation step and the second step comprises a measurement step.

6. The medical system of any of claims 1-5, wherein, to determine the cumulative amount of contrast used, the processing circuitry (2004) is configured to:
receive, via a user interface, a manual entry of the cumulative amount of contrast used.

7. The medical system of any of claims 1-5, further comprising:
a wireless communication unit;
wherein, to determine the cumulative amount of contrast used, the processing circuitry (2004) is configured to:
receive, from a flow meter via the wireless communication unit, data that represents the cumulative amount of contrast used.

8. The medical system of any of claims 1-7, wherein the graphical representation of the cumulative amount of contrast used comprises one or more of:
a graph that illustrates the cumulative amount of contrast used, wherein the graph comprises a plot of an amount of contrast used over time; and
a textual representation of the cumulative amount of contrast used.

9. The medical system of any of claims 1-8, wherein the processing circuitry (2004) is further configured to:
obtain, for a current patient, a maximum contrast dosage for the cardiac catheterization lab procedure (5000); and
output, for display (2006) and during the cardiac catheterization lab procedure (5000), a graphical representation of a comparison between the cumulative amount of contrast used and the maximum contrast dosage.

10. The medical system of any of claims 1-9, further comprising:
an injector configured to inject contrast into the patient,
wherein the processing circuity is further configured to control the injector.

11. The medical system of claim 10, wherein, to control the injector, the processing circuitry (2004) is configured to control the injector based on the cumulative amount of contrast used.

12. The medical system of any preceding claim, wherein the imaging data captured prior to the cardiac catheterization lab procedure (5000) comprises diagnostic angiogram imaging data.

## Patentansprüche

1. Medizinisches System, umfassend:
einen Speicher (2002); und
eine Verarbeitungsschaltung (2004), die mit dem Speicher (2002) kommunikativ gekoppelt ist, wobei die Verarbeitungsschaltung (2004) konfiguriert ist zum:
Bestimmen, zu einem aktuellen Zeitpunkt eines Herzkatheterlaboreingriffs, einer kumulativen Menge an Kontrastmittel, die während des Herzkatheterlaboreingriffs (5000) bis zu dem aktuellen Zeitpunkt verwendet wurde;
Bestimmen einer vorhergesagten Menge an Kontrastmittel, die bis zu dem aktuellen Zeitpunkt des Herzkatheterlaboreingriffs verwendet worden sein soll, wobei, um die vorhergesagte Menge an Kontrastmittel zu bestimmen, die Verarbeitungsschaltung konfiguriert ist zum:
Bestimmen, basierend auf den Bildgebungsdaten des aktuellen Patienten, die vor dem Herzkatheterlaboreingriff (5000) erfasst wurden, einer Klassifizierung des aktuellen Patienten; und
Bestimmen, basierend auf der Menge an Kontrastmittel, die für andere Patienten verwendet werden, die die Klassifizierung aufweisen, der vorhergesagten Menge an Kontrastmittel für den aktuellen Patienten; und
Ausgeben, für eine Anzeige (2006) und während des Herzkatheterlaboreingriffs (5000), einer grafischen Darstellung der kumulativen Menge an Kontrastmittel, die verwendet wurde, und der vorhergesagten Menge an Kontrastmittel, die verwendet worden sein soll.

2. Medizinisches System nach Anspruch 1, wobei der Herzkatheterlaboreingriff (5000) eine Vielzahl von Schritten umfasst, und wobei die Verarbeitungsschaltung (2004) ferner konfiguriert ist zum:
Bestimmen einer Zielkontrastdosis für einen ersten Schritt der Vielzahl von Schritten;
Bestimmen einer Menge an Kontrastmittel, die während einer Durchführung des ersten Schritts verwendet wird; und
Ausgeben, für die Anzeige (2006) und während des Herzkatheterlaboreingriffs (5000), einer grafischen Darstellung eines Vergleichs zwischen der Zielkontrastmitteldosis für den ersten Schritt und der Menge an Kontrastmittel, die während der Durchführung des ersten Schritts verwendet wird.

3. Medizinisches System nach Anspruch 2, wobei die Verarbeitungsschaltung (2004) ferner konfiguriert ist zum:
Bestimmen einer Zielkontrastdosis für einen zweiten Schritt der Vielzahl von Schritten, wobei sich die Zielkontrastdosis für den zweiten Schritt von der Zielkontrastdosis für den ersten Schritt unterscheidet;
Bestimmen einer Menge an Kontrastmittel, die während der Durchführung des zweiten Schritts verwendet wird; und
Ausgeben, für die Anzeige (2006) und während des Herzkatheterlaboreingriffs (5000), einer grafischen Darstellung eines Vergleichs zwischen der Zielkontrastmitteldosis für den zweiten Schritt und der Menge an Kontrastmittel, die während der Durchführung des zweiten Schritts verwendet wird.

4. Medizinisches System nach Anspruch 3, wobei die Zielkontrastdosis für den zweiten Schritt größer als die Zielkontrastdosis für den ersten Schritt ist.

5. Medizinisches System nach Anspruch 4, wobei der erste Schritt einen Navigationsschritt umfasst und der zweite Schritt einen Messschritt umfasst.

6. Medizinisches System nach einem der Ansprüche 1 bis 5, wobei, um die kumulative Menge an Kontrastmittel, die verwendet wird, zu bestimmen, die Verarbeitungsschaltung (2004) konfiguriert ist zum:
Empfangen, über eine Benutzerschnittstelle, einer manuellen Eingabe der kumulativen Menge an Kontrastmittel, die verwendet wird.

7. Medizinisches System nach einem der Ansprüche 1 bis 5, ferner umfassend:
eine drahtlose Kommunikationseinheit;
wobei, um die kumulative Menge an Kontrastmittel, die verwendet wird, zu bestimmen die Verarbeitungsschaltung (2004) konfiguriert ist zum:
Empfangen, von einem Durchflussmesser über die drahtlose Kommunikationseinheit, von Daten, die die kumulative Menge an Kontrastmittel, die verwendet wird, darstellen.

8. Medizinisches System nach einem der Ansprüche 1 bis 7, wobei die grafische Darstellung der kumulativen Menge an Kontrastmittel, die verwendet wird, eines oder mehrere umfasst von:
einem Diagramm, das die kumulative Menge an Kontrastmittel veranschaulicht, die verwendet wird, wobei das Diagramm eine grafische Darstellung einer Menge an Kontrastmittel, die im Laufe der Zeit verwendet wird, umfasst; und
einer textuellen Darstellung der kumulativen Menge an Kontrastmittel, die verwendet wird.

9. Medizinisches System nach einem der Ansprüche 1 bis 8, wobei die Verarbeitungsschaltung (2004) ferner konfiguriert ist zum:
Erhalten, für einen aktuellen Patienten, einer maximalen Kontrastmitteldosis für den Herzkatheterlaboreingriff (5000); und
Ausgeben, für die Anzeige (2006) und während des Herzkatheterlaboreingriffs (5000), einer grafischen Darstellung eines Vergleichs zwischen der kumulativen Menge an Kontrastmittel, die verwendet wird, und der maximalen Kontrastmitteldosis.

10. Medizinisches System nach einem der Ansprüche 1 bis 9, ferner umfassend:
einen Injektor, der konfiguriert ist, um Kontrastmittel in den Patienten zu injizieren,
wobei die Verarbeitungsschaltung ferner konfiguriert ist, um den Injektor zu steuern.

11. Medizinisches System nach Anspruch 10, wobei, um den Injektor zu steuern, die Verarbeitungsschaltung (2004) konfiguriert ist, um den Injektor basierend auf der kumulativen Menge an Kontrastmittel, die verwendet wird, zu steuern.

12. Medizinisches System nach einem der vorstehenden Ansprüche, wobei die Bildgebungsdaten, die vor dem Herzkatheterlaboreingriff (5000) erfasst wurden, Bildgebungsdaten eines diagnostische Angiogramms umfassen.

## Revendications

1. Système médical comprenant :
une mémoire (2002) ; et
une circuiterie de traitement (2004) couplée de manière communicative à la mémoire (2002), la circuiterie de traitement (2004) étant configurée pour :
déterminer, à un moment actuel d'une procédure de laboratoire de cathétérisme cardiaque, une quantité cumulée de contraste utilisée pendant la procédure de laboratoire de cathétérisme cardiaque (5000) jusqu'au moment actuel ;
déterminer une quantité prédite de contraste devant avoir été utilisée au moment actuel de la procédure de laboratoire de cathétérisme cardiaque, dans lequel, pour déterminer la quantité prédite de contraste, la circuiterie de traitement est configurée pour :
déterminer, sur la base des données d'imagerie du patient actuel capturées avant la procédure de laboratoire de cathétérisme cardiaque (5000), une classification du patient actuel ; et
déterminer, sur la base de la quantité de contraste utilisée pour d'autres patients ayant la classification, la quantité prédite de contraste pour le patient actuel ; et
fournir en sortie, pour affichage (2006) et pendant la procédure de laboratoire de cathétérisme cardiaque (5000), une représentation graphique de la quantité cumulée de contraste utilisée et de la quantité prédite de contraste devant avoir été utilisée.

2. Système médical selon la revendication 1, dans lequel la procédure de laboratoire de cathétérisme cardiaque (5000) est constituée d'une pluralité d'étapes, et dans lequel la circuiterie de traitement (2004) est en outre configurée pour :
déterminer un dosage de contraste cible pour une première étape de la pluralité d'étapes ;
déterminer une quantité de contraste utilisée lors de l'exécution de la première étape ; et
fournir en sortie, pour affichage (2006) et pendant la procédure de laboratoire de cathétérisme cardiaque (5000), une représentation graphique d'une comparaison entre le dosage de contraste cible pour la première étape et la quantité de contraste utilisée pendant l'exécution de la première étape.

3. Système médical selon la revendication 2, dans lequel la circuiterie de traitement (2004) est en outre configurée pour :
déterminer un dosage de contraste cible pour une seconde étape de la pluralité d'étapes, le dosage de contraste cible pour la seconde étape étant différent du dosage de contraste cible pour la première étape ;
déterminer une quantité de contraste utilisée lors de l'exécution de la seconde étape ; et
fournir en sortie, pour affichage (2006) et pendant la procédure de laboratoire de cathétérisme cardiaque (5000), une représentation graphique d'une comparaison entre le dosage de contraste cible pour la seconde étape et la quantité de contraste utilisée pendant l'exécution de la seconde étape.

4. Système médical selon la revendication 3, dans lequel le dosage de contraste cible pour la seconde étape est supérieur au dosage de contraste cible pour la première étape.

5. Système médical selon la revendication 4, dans lequel la première étape comprend une étape de navigation et la seconde étape comprend une étape de mesure.

6. Système médical selon l'une quelconque des revendications 1 à 5, dans lequel, pour déterminer la quantité cumulée de contraste utilisée, la circuiterie de traitement (2004) est configurée pour :
recevoir, par l'intermédiaire d'une interface utilisateur, une saisie manuelle de la quantité cumulée de contraste utilisée.

7. Système médical selon l'une quelconque des revendications 1 à 5, comprenant en outre :
une unité de communication sans fil ;
dans lequel, pour déterminer la quantité cumulée de contraste utilisée, la circuiterie de traitement (2004) est configurée pour :
recevoir, en provenance d'un débitmètre par l'intermédiaire de l'unité de communication sans fil, des données représentant la quantité cumulée de contraste utilisée.

8. Système médical selon l'une quelconque des revendications 1 à 7, dans lequel la représentation graphique de la quantité cumulée de contraste utilisée comprend un ou plusieurs parmi :
un graphique qui illustre la quantité cumulée de contraste utilisée, dans lequel le graphique comprend un tracé d'une quantité de contraste utilisée au fil du temps ; et
une représentation textuelle de la quantité cumulée de contraste utilisée.

9. Système médical selon l'une quelconque des revendications 1 à 8, dans lequel la circuiterie de traitement (2004) est en outre configurée pour :
obtenir, pour un patient actuel, un dosage maximal de contraste pour la procédure de laboratoire de cathétérisme cardiaque (5000) ; et
fournir en sortie, pour un affichage (2006) et pendant la procédure de laboratoire de cathétérisme cardiaque (5000), une représentation graphique d'une comparaison entre la quantité cumulée de contraste utilisée et le dosage maximal de contraste.

10. Système selon l'une quelconque des revendications 1 à 9, comprenant en outre :
un injecteur configuré pour injecter du contraste dans le patient,
dans lequel la circuiterie de traitement est en outre configurée pour commander l'injecteur.

11. Système médical selon la revendication 10, dans lequel, pour commander l'injecteur, la circuiterie de traitement (2004) est configurée pour commander l'injecteur sur la base de la quantité cumulée de contraste utilisée.

12. Système médical selon l'une quelconque des revendications précédentes, dans lequel les données d'imagerie capturées avant la procédure de laboratoire de cathétérisme cardiaque (5000) comprennent des données d'imagerie d'angiogramme à visée diagnostique.
